# EUROPEAN PATENT SPECIFICATION

(11) **EP 3 142 683 B1**
(45) Date of publication and mention of the grant of the patent: **20.02.2019**
(21) Application number: 15727759.1
(22) Date of filing: 15.05.2015
(51) Int. Cl.: A61K 38/16

(54) **ANTI-INFLAMMATORY COMPOUNDS AND METHODS OF USE**
ENTZÜNDUNGSHEMMENDE VERBINDUNGEN UND VERFAHREN ZUR VERWENDUNG
COMPOSÉS ANTI-INFLAMMATOIRES ET LEURS MÉTHODES D'UTILISATION

(30) Priority: 16.05.2014 US 201461994601 P
(43) Date of publication of application: 22.03.2017
(73) Proprietor: University Of Oregon, Eugene, Oregon 97403 (US)
(72) Inventor: GUILLEMIN, Karen, Eugene, OR 97403-1229 (US); ROLIG, Annah, Eugene, OR 97403-1229 (US)
(74) Representative: Mewburn Ellis LLP
(86) International application number: PCT/US2015/031052
(87) International publication number: WO 2015/175919

(56) References cited:
- WO-A2-01/34175
- WO-A2-2011/160062
- L. BOMAR ET AL: "Draft Genome Sequence of Aeromonas veronii Hm21, a Symbiotic Isolate from the Medicinal Leech Digestive Tract", GENOME ANNOUNCEMENTS, vol. 1, no. 5, 3 October 2013 (2013-10-03) , pages e00800-13, XP055202485, ISSN: 2169-8287, DOI: 10.1128/genomeA.00800-13 & DATABASE NCBI Reference Sequence [Online] NCBI; 21 September 2013 (2013-09-21), "hypothetical protein [Aeromonas veronii]", retrieved from NCBI Database accession no. WP_021230730
- JENNIFER M. BATES ET AL: "Intestinal Alkaline Phosphatase Detoxifies Lipopolysaccharide and Prevents Inflammation in Zebrafish in Response to the Gut Microbiota", CELL HOST & MICROBE, vol. 2, no. 6, 1 December 2007 (2007-12-01), pages 371-382, XP055202486, ISSN: 1931-3128, DOI: 10.1016/j.chom.2007.10.010

### SEQUENCE LISTING

<110> University of Oregon Guillemin, Karen Rolig, Annah
<120> ANTI-INFLAMMATORY COMPOUNDS AND METHODS OF USE
<130> 1505-92355-02
<150> US 61/994601
   <151> 2014-05-16
<160> 10
<170> PatentIn version 3.5
<210> 1
   <211> 313
   <212> PRT
   <213> Aeromonas veronii
<400> 1
   Met Lys Met His Asn Lys Ala Leu Leu Ala Ile Ala Cys Ala Ala Ala
   1               5                   10                  15
   Leu Ser Ala Cys Asp Ser Glu Lys Ala Ala Asn Ser Ile Ala Lys Arg
               20                  25                  30
   Val Pro Leu Ala Leu Pro Glu Ala Gly Leu Tyr Gln Ala Asn Leu Met
           35                  40                  45
   Ser Arg Asp Gly Asp Lys Ala Thr Pro Arg Met Ile Lys Asp Leu Asp
       50                  55                  60
   Gly Leu Ala Leu Val Tyr Pro Lys Gly Glu Thr Val Gln His Trp Gly
   65                  70                  75                  80
   Val Trp Val Asp His Gln Val Gly Lys Val Glu Thr Asn Ser Gln Trp
                   85                  90                  95
   Leu Gly Gln Ala Asp Gln Lys Ala Asp Lys Asp Gly Ile Tyr Pro Val
               100                 105                 110
   Gln Leu Ile Arg Asn Ser Glu Arg Leu Gly Thr Ser Thr Ala Leu Ser
           115                 120                 125
   Ser Val Thr Asn Asp His Asn Leu Ile Thr Phe Gln Asp Gln Pro Val
       130                 135                 140
   Ile Asp Leu Gln Gly Lys Glu Ile Lys Arg Trp Val Phe Asp Phe Thr
   145                 150                 155                 160
   Arg Thr Gly Thr Lys Phe Ser Asp Asn Ser Pro Ile Tyr Ser Gly Phe
                   165                 170                 175
   Ser Gly His Val Ala Val Thr Ala Leu Thr Thr Lys Ala Val Thr Thr
               180                 185                 190
   Ala Ser Trp Ser Ala Thr Asp Ser Asp Gly Phe Ser Ser Glu Met Val
           195                 200                 205
   Gly Lys Val Asp Thr Thr Asn Asn Gly Gly Lys Leu Thr Val Ala Ile
       210                 215                 220
   Glu Phe Pro Ala Ala Gly Cys Thr Leu Val Gly Glu Gly Ser Ala Thr
   225                 230                 235                 240
   Ala Gly Leu Ser Lys Leu Thr Met Thr Gly Phe Gly Lys Cys Asn Phe
                   245                 250                 255
   Lys Gln Ser Ala Ala Ala Thr Pro Ile Glu Asn Leu Trp Asn Ala Ala
               260                 265                 270
   Leu Ala Arg Ala Met Asp Asn Arg Val Ala Tyr Val Thr Thr Phe Thr
           275                 280                 285
   Ala Asp Ala Lys Lys Glu Ala Leu Val Ile Gly Phe Pro Asp Thr Asn
       290                 295                 300
   Gly Leu Leu Ile Thr Ala Asp Lys Arg
   305                 310
<210> 2
   <211> 942
   <212> DNA
   <213> Aeromonas veronii
<400> 2
   atgaaaatgc acaacaaagc gctgctggcc atcgcctgcg ccgccgccct gtctgcctgt      60
   gacagcgaaa aagctgccaa cagcattgcc aagcgggtgc cgctggctct gcctgaggcg     120
   ggtctttacc aagcgaatct gatgagccgc gacggcgaca aggcgacgcc gcgcatgatc     180
   aaggatctgg atggtctggc gctggtctac ccgaagggtg aaaccgtgca gcactggggt     240
   gtctgggtgg atcatcaggt gggcaaggtc gagaccaaca gccagtggtt ggggcaggct     300
   gaccagaaag ccgacaaaga cgggatctac ccggtgcagt tgatccgcaa cagcgaacgg     360
   ctcggcacca gcactgcgct cagctcggtg accaacgacc acaacctcat caccttccag     420
   gatcagccgg tcatcgatct gcagggcaag gagatcaagc gctgggtttt tgatttcacc     480
   cggactggca ccaagttctc cgacaactcc cccatctatt ccggtttcag cggtcatgtg     540
   gcggtgacgg ccctgaccac caaagcggtg accacggcca gctggagtgc cacggacagc     600
   gatggcttca gcagcgaaat ggtgggcaag gtggatacca ccaacaacgg cggcaagctg     660
   acggtagcga tcgagtttcc ggcggcaggt tgtacgctgg tgggcgaggg gagtgccact     720
   gcagggttga gtaagctgac catgaccggg tttggcaagt gcaacttcaa gcaatccgct     780
   gcagccacgc cgatcgaaaa cctctggaat gctgctctgg cccgggcgat ggataacaga     840
   gtcgcctatg tcaccacctt taccgcggat gccaaaaaag aggcgctggt catcggcttc     900
   cccgacacca atggcttgct gatcaccgct gacaagcgat aa                        942
<210> 3
   <211> 35
   <212> DNA
   <213> Artificial Sequence
<220>
   <223> Synthetic oligonucleotide - AP forward primer
<400> 3
   cgtacatatg atgaaaatgc acaacaaagc gctgc 35
<210> 4
   <211> 34
   <212> DNA
   <213> Artificial Sequence
<220>
   <223> Synthetic oligonucleotide - AP reverse primer
<400> 4
   ctgactcgag ttatcgcttg tcagcggtga tcag 34
<210> 5
   <211> 23
   <212> DNA
   <213> Artificial Sequence
<220>
   <223> Synthetic oligonucleotide primer
<400> 5
   gcgacagcaa ggaataaaaa ctc 23
<210> 6
   <211> 23
   <212> DNA
   <213> Artificial Sequence
<220>
   <223> Synthetic oligonucleotide primer
<400> 6
   cacccctgcc gttagctgct tat 23
<210> 7
   <211> 23
   <212> DNA
   <213> Artificial Sequence
<220>
   <223> Synthetic oligonucleotide primer
<400> 7
   ctaaggagga tattcatatg cat 23
<210> 8
   <211> 22
   <212> DNA
   <213> Artificial Sequence
<220>
   <223> Synthetic oligonucleotide primer
<400> 8
   gatggtctgg gtattgccgt tg 22
<210> 9
   <211> 22
   <212> DNA
   <213> Artificial Sequence
<220>
   <223> Synthetic oligonucleotide primer
<400> 9
   cgaagcagct ccagcctaca ca 22
<210> 10
   <211> 22
   <212> DNA
   <213> Artificial Sequence
<220>
   <223> Synthetic oligonucleotide primer
<400> 10
   gctgttcgtc atcgatcggc gc 22

## Description

### FIELD

This disclosure relates to anti-inflammatory proteins from gut microbiota and methods of their use.

### BACKGROUND

Inflammatory disorders such as allergies, asthma, rheumatoid arthritis, inflammatory bowel disease, and atherosclerosis affect large numbers of people. While anti-inflammatory drugs are available, many have serious side effects, including increased risk of stroke or gastric damage or are systemic suppressors of the immune system. Thus, there remains a need for additional anti-inflammatory therapies.

L. Bomar et al. (Genome Announcements, 2013; 1(5): 1-2) disclose a draft genome sequence of *Aeromonas veronii* Hm21, a symbiotic isolate from the medicinal leech digestive tract.

WO 01/34175 A2 provides a method of treating or preventing inflammation in a subject, comprising administering to the subject an effective amount of cholera toxin subunit B.

WO 2011/160062 A2 provides methods and compositions for treating inflammatory bowel disease, including ulcerative colitis and Crohn's Disease, and other related conditions, by locally administering to the intestinal mucosa of a subject having inflammatory bowel disease a therapeutically effective amount of IL-27 or a therapeutic variant or fragment thereof.

J.M Bates et al. (Cell Host & Microbe, 2007; 2(6): 371-382) discuss intestinal alkaline phosphatase detoxifying lipopolysaccharide and preventing inflammation in zebrafish in response to the gut microbiota.

### SUMMARY

The inventors have identified a protein from *Aeromonas* with anti-inflammatory activity (referred to herein in some examples as anti-inflammatory protein (AP) or protein 1882). Disclosed herein are methods of treating or inhibiting (for example, reducing) inflammation in a subject by administering the newly identified anti-inflammatory protein to a subject. In some embodiments, the protein has at least 80% sequence identity to the amino acid sequence set forth as SEQ ID NO: 1 or fragments thereof and is for use in a method of treating or inhibiting inflammation in a subject. In some examples, the subject has an inflammatory disease, such as inflammatory bowel disease, rheumatoid arthritis, osteoarthritis, inflammatory lung disease, atherosclerosis, systemic lupus erythematosus, Sjogren's syndrome, asthma, allergic rhinitis, psoriasis, irritable bowel syndrome, necrotizing enterocolitis, or atopy.

Recombinant vectors including a nucleic acid encoding the herein identified anti-inflammatory protein (such as a nucleic acid encoding a protein with at least 80% sequence identity to SEQ ID NO: 1 or a fragment thereof) operably linked to a heterologous promoter are also disclosed. In some examples, the nucleic acid encoding the protein is set forth in SEQ ID NO: 2. Cells including the recombinant vector (for example, cells transformed with the vector) are also disclosed.

The foregoing and other features of the disclosure will become more apparent from the following detailed description, which proceeds with reference to the accompanying figures.

### BRIEF DESCRIPTION OF THE DRAWINGS

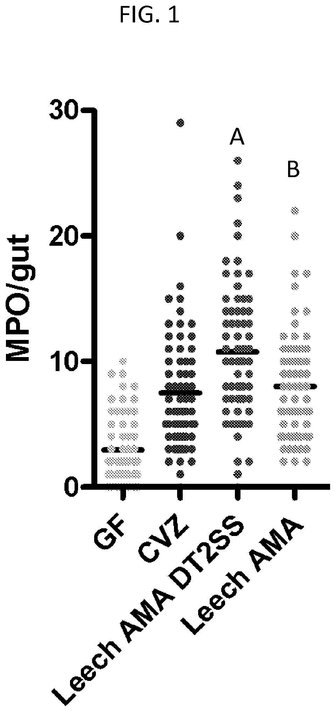

FIG. 1 is a graph showing the number of myeloperoxidase (MPO) positive cells in the gut of zebrafish raised germ-free (GF), conventionally (CVZ), mono-associated with type II secretion system (T2SS) deletion mutant *A. veronii* (Leech AMA DT2SS), and mono-associated with wild-type (WT) *Aeromonas veronii* (Leech AMA). A and B indicate groups that are significantly different from one another (ANOVA, p<0.05).

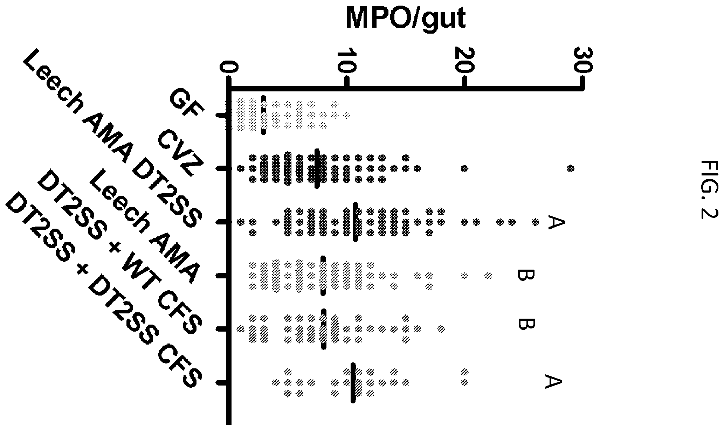

FIG. 2 is a graph showing the number of MPO positive cells in the gut of zebrafish raised GF, CVZ, mono-associated with DT2SS *A. veronii* (Leech AMA DT2SS), mono-associated with WT *A. veronii* (Leech AMA), or mono-associated with DT2SS *A. veronii* and cell-free supernatant (CFS) from WT *A. veronii* (DT2SS + WT CFS) or from DT2SS *A. veronii* (DT2SS + DT2SS CFS). A and B indicate groups that are significantly different from one another (ANOVA, p<0.05). The first four lanes repeat data shown in FIG. 1, for the sake of comparison.

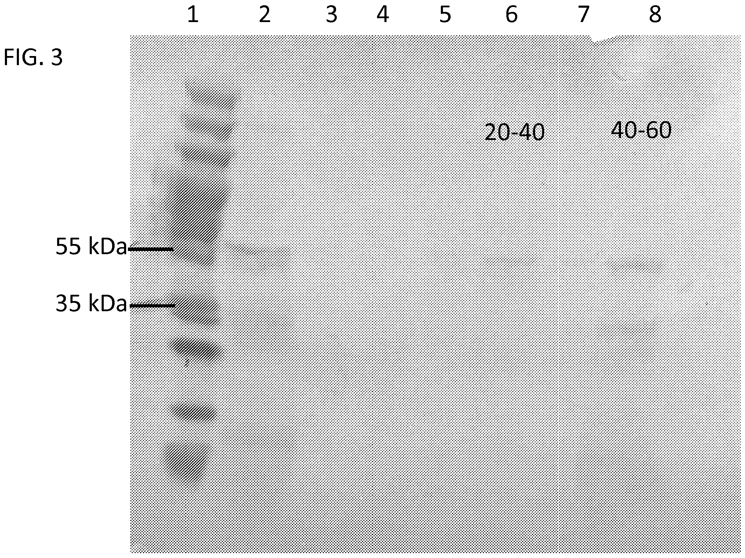

FIG. 3 is a digital image of Coomassie-blue stained SDS-PAGE of *A. veronii* CFS fractions. Lane 1: ladder; lane 2: concentrated CFS; lane 3: empty; lane 4: 0-20% ammonium sulfate fraction; lane 5: empty; lane 6: 20-40% ammonium sulfate fraction; lane 7: empty; lane 8: 40-60% ammonium sulfate fraction.

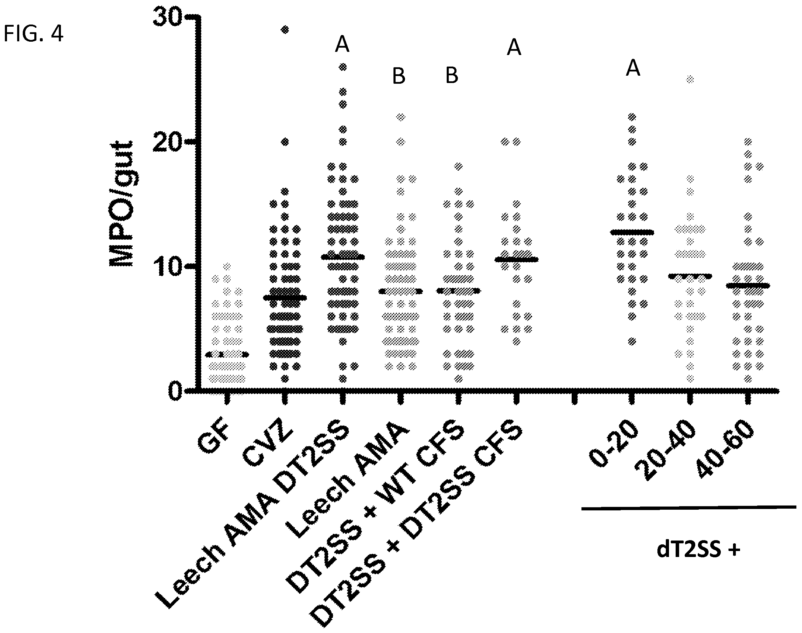

FIG. 4 is a graph showing the number of MPO positive cells in the gut of zebrafish raised GF, CVZ, mono-associated with DT2SS *A. veronii* (Leech AMA DT2SS), mono-associated with WT *A. veronii* (Leech AMA), mono-associated with DT2SS *A. veronii* and CFS from WT *A. veronii* (DT2SS + WT CFS), mono-associated with DT2SS *A. veronii* and CFS from DT2SS *A. veronii* (DT2SS + DT2SS CFS), mono-associated with DT2SS *A. veronii* plus 0-20% ammonium sulfate fraction, (0-20), mono-associated with DT2SS *A. veronii* plus 20-40% ammonium sulfate fraction (20-40), or mono-associated with DT2SS *A. veronii* plus 40-60% ammonium sulfate fraction (40-60). A and B indicate groups that are significantly different (ANOVA, p<0.05). The first six lanes repeat data shown in FIG. 2, for the sake of comparison.

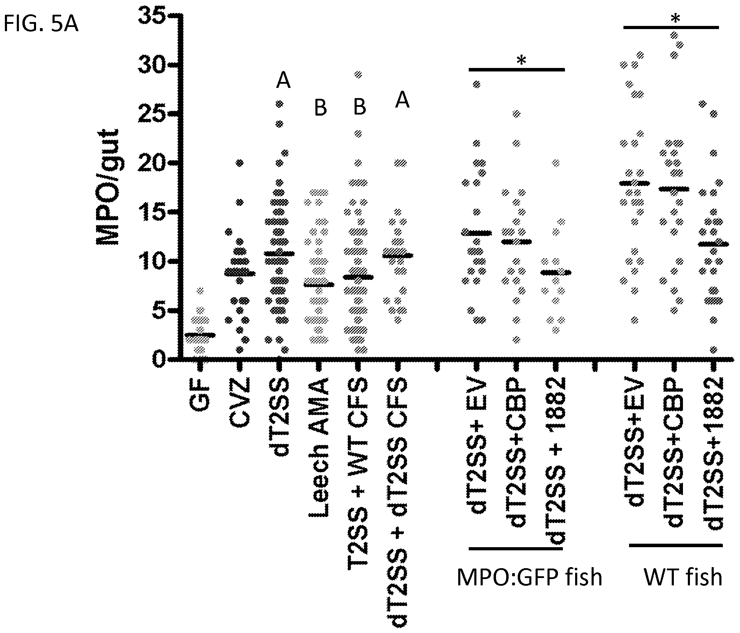

FIG. 5A is a graph showing the number of MPO positive cells in the gut of zebrafish raised GF, CVZ, mono-associated with DT2SS *A. veronii* (Leech AMA DT2SS), mono-associated with WT *A. veronii* (Leech AMA), mono-associated with DT2SS *A. veronii* plus CFS from WT *A. veronii* (DT2SS + WT CFS), or mono-associated with DT2SS *A. veronii* plus CFS from DT2SS *A. veronii* (DT2SS + DT2SS CFS). The graph also shows zebrafish transgenic for MPO:GFP or WT fish mono-associated with DT2SS *A. veronii* plus CFS from *E. coli* carrying a control plasmid (DT2SS + EV), mono-associated with DT2SS *A. veronii* plus CFS from *E. coli* carrying a plasmid for chitin binding protein (dT2SS + CBP), or mono-associated with DT2SS *A. veronii* plus CFS from *E. coli* carrying a plasmid with the gene for AP (dT2SS + 1882). A and B indicate groups that are significantly different (ANOVA, p<0.05).
   * indicates significantly different between +EV condition and +AP (1882) condition.

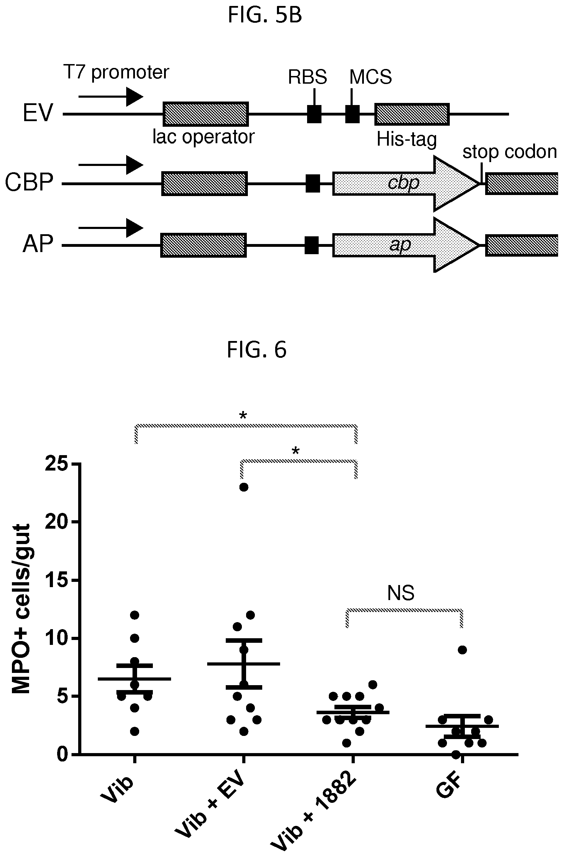

FIG. 5B is a diagram showing the *E. coli* expression vectors for AP and CBP proteins. Expression is induced with IPTG. RBS, ribosome binding site; MCS, multiple cloning site; EV, empty vector.
FIG. 6 is a graph showing the number of MPO positive cells in the gut of zebrafish infected with *Vibrio* (Vib: 48 hours of *Vibrio* infection), zebrafish infected with *Vibrio* treated with CFS from *E. coli* carrying a control plasmid (Vib + EV), zebrafish infected with *Vibrio* treated with CFS from *E. coli* carrying a plasmid with the gene for AP under an inducible promoter (Vib + 1882), or GF. *: p < 0.05; NS: not significant.

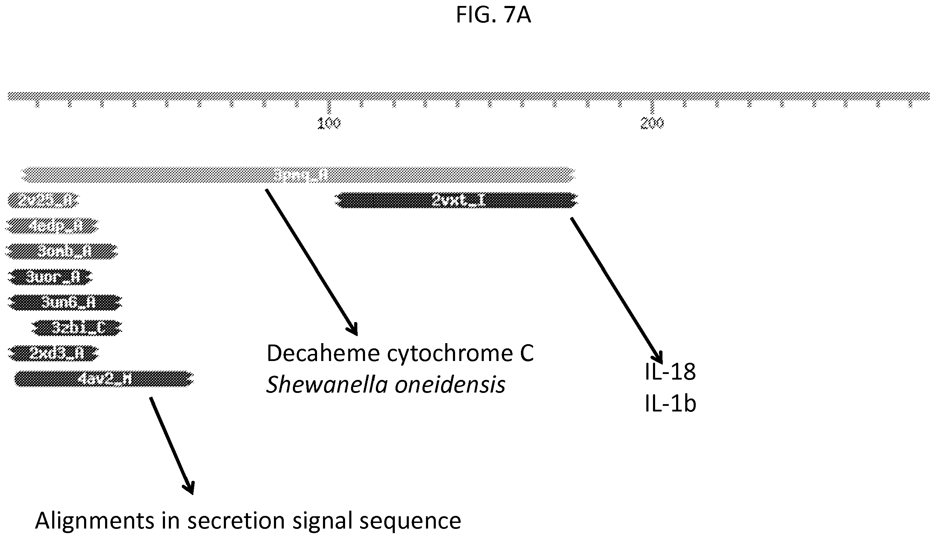

FIG. 7A is a schematic showing *A. veronii* AP (hatched line) and conserved structural domains compared to known proteins. The N-terminal region of *A. veronii* AP shows similarity to secretion signal sequences from other proteins. The main conserved domain (3pnq_A) has similarity to decaheme cytochrome C from *Shewanella oneidensis.* The protein also includes a beta-trefoil domain (2vxt_I) that is also found in the cytokine superfamily (such as IL-18 and IL-1b).

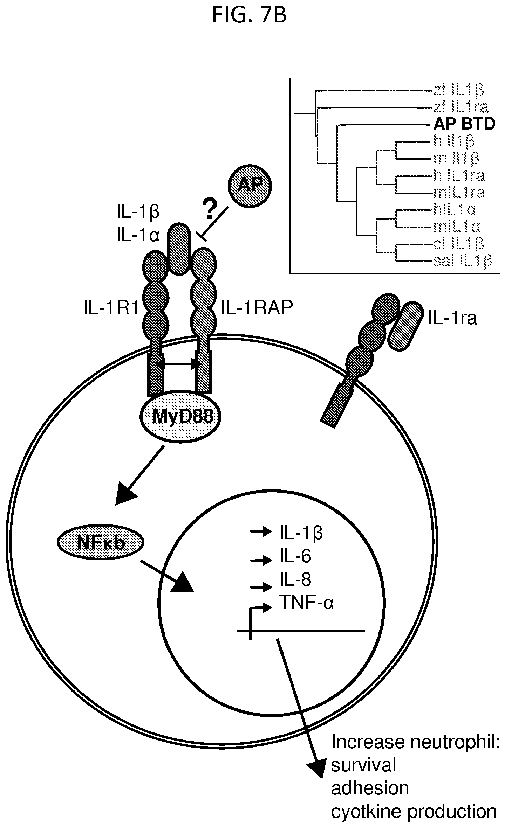

FIG. 7B is a diagram illustrating interleukin-1 (IL-1) signaling pathway and a proposed role for AP in blocking IL-1R1 signaling. The inset shows phylogenetic relationship of the AP β-trefoil domain (BTD) to the same domain from the indicated human (h), mouse (m), zebrafish (z), salmon (sal), and catfish (cf) proteins. The sequences used in constructing the phylogenetic tree are as follows (GenBank Accession Nos.): zfIL1β, NP_998009.2; zfIL1ra, AEJ36293.1; hIL1β, NP_000567.1; mIL1β, AAH11437.1; hIL1ra, AAH09745.1; mIL1ra, AAA39278.1; hILIα, CAG33695.1; mIL1α, AAH03727.1; cfIL1β, NP_001187149; and salIL1β, NP_0011178191. AP BTD used in generating the phylogenetic tree was amino acids 148-176 of SEQ ID NO: 1.

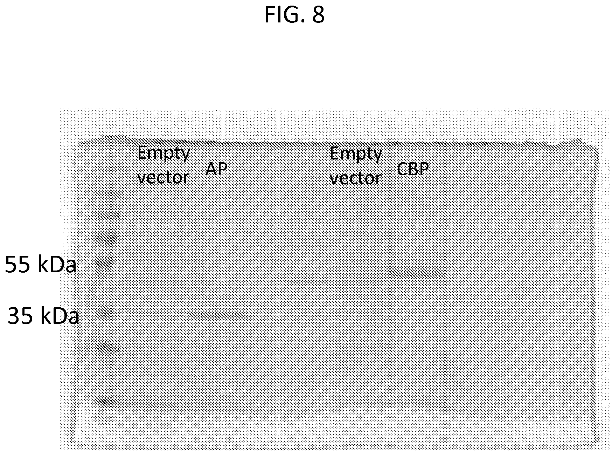

FIG. 8 is a digital image of a Coomassie-blue stained gel showing supernatant from cultures of *E. coli* carrying a control plasmid (empty vector) or a plasmid with the AP gene (AP) or the chitin binding protein gene (CBP).

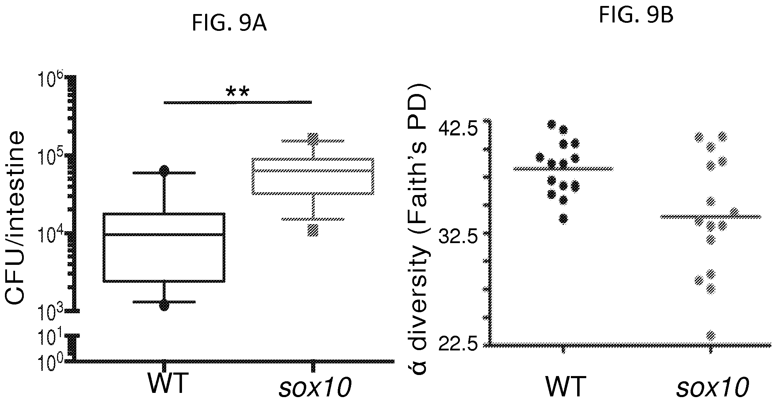

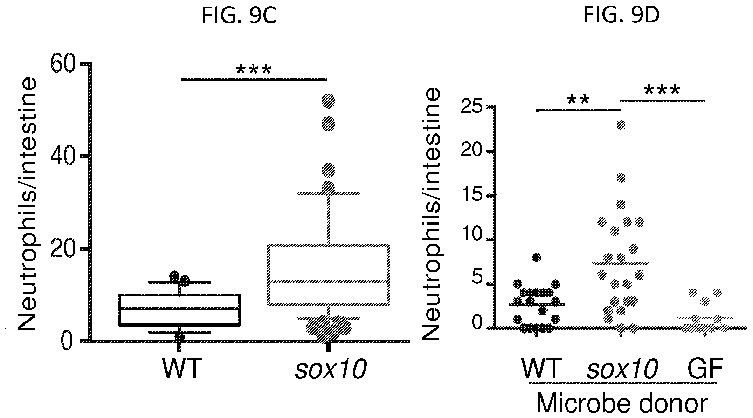

FIGS. 9A-9D are a series of graphs showing *sox10* zebrafish characteristics and the effect of inoculating germ-free fish with microbiota from *sox10* fish. FIG. 9A is a graph showing bacterial load in WT and *sox10* fish. FIG. 9B is a graph showing diversity of the bacterial communities in WT and *sox10* fish. FIG. 9C is a graph showing neutrophil influx to the intestine in WT and *sox10* fish. FIG. 9D is a graph showing neutrophil influx to the intestine in WT GF zebrafish inoculated with either microbiota from a WT or a *sox10* donor fish. **p<0.01, ***p<0.001, T test.

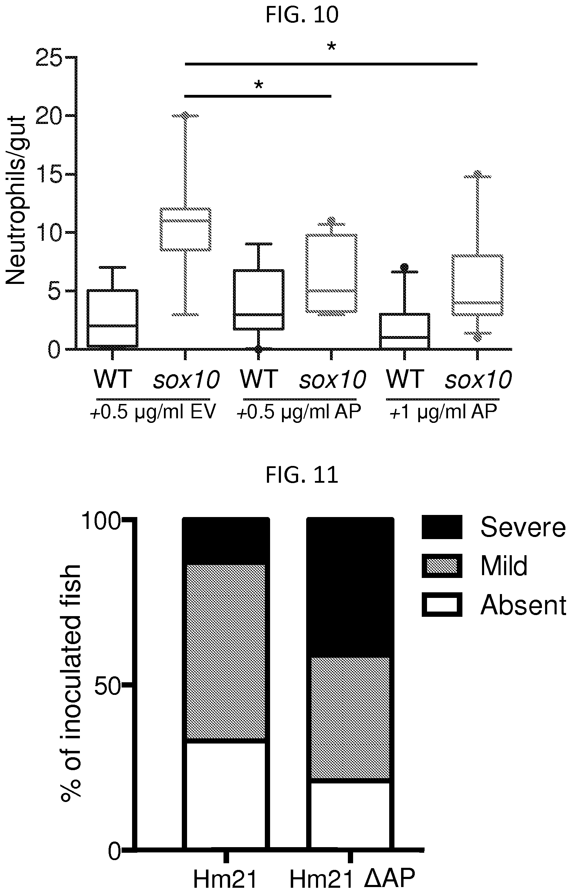

FIG. 10 is a graph showing neutrophil influx in WT or *sox10* zebrafish treated with empty vector (EV), 0.5 µg/ml CFS from *E. coli* expressing AP protein, or 1 µg/ml CFS from *E. coli* expressing AP protein. *p<0.05.
FIG. 11 is a graph showing the effect of infection of WT zebrafish with *A. veronii* Hm21 or Hm21 ΔAP on inflammation (neutrophil influx in the intestine). The graph shows percent of inoculated fish with severe (black), mild (gray), or no (white) inflammation.

### SEQUENCE LISTING

The nucleic and amino acid sequences provided herein or in the accompanying Sequence Listing are shown using standard letter abbreviations for nucleotide bases and amino acids. Only one strand of each nucleic acid sequence is shown, but the complementary strand is understood as included by any reference to the displayed strand.
SEQ ID NO: 1 is an exemplary amino acid sequence of *A. veronii* AP.
SEQ ID NO: 2 is an exemplary nucleic acid sequence encoding *A. veronii* AP.
SEQ ID NOs: 3 and 4 are forward and reverse primers, respectively, for amplification of an *A. veronii* AP nucleic acid.
SEQ ID NOs: 5 and 6 are forward and reverse primers, respectively for amplification of a chloramphenicol resistance cassette.
SEQ ID NOs: 7-10 are primers used for amplification of an approximately 1000 base pair region upstream and downstream of the AP gene in *A. veronii* Hm21.

### DETAILED DESCRIPTION

### I. Abbreviations

- **AP**: bacterial anti-inflammatory protein (also referred to as protein 1882)
- **BTD**: beta-trefoil domain
- **CFS**: cell-free supernatant
- **CV**: conventionally reared
- **DPF**: days post-fertilization
- **DT2SS** or **ΔT2SS**: type II secretion system deletion
- **GF**: germ-free reared
- **GFP**: green fluorescent protein
- **IBD**: inflammatory bowel disease
- **MPO**: myeloperoxidase
- **T2SS**: type II secretion system
- **WT**: wild type

### II. Terms

Unless otherwise noted, technical terms are used according to conventional usage. Unless otherwise explained, all technical and scientific terms used herein have the same meaning as commonly understood by one of ordinary skill in the art to which this disclosure belongs. The singular terms "a," "an," and "the" include plural referents unless context clearly indicates otherwise. Although methods and materials similar or equivalent to those described herein can be used in the practice or testing of the present disclosure, suitable methods and materials are described below.

In case of conflict, the present specification, including explanations of terms, will control. In addition, the materials, methods, and examples are illustrative only and not intended to be limiting.

In order to facilitate review of the various embodiments of the disclosure, the following explanations of specific terms are provided:
**Effective amount:** An amount of an agent or composition that alone, or together with a pharmaceutically acceptable carrier and/or one or more additional agents, induces the desired response. Effective amounts of an agent can be determined in many different ways, such as assaying for a reduction in neutrophil recruitment, delay (or even prevention) of onset of a condition associated with inflammation, or a reduction or amelioration of one or more symptoms of a subject with inflammation. Effective amounts also can be determined through various *in vitro*, *in vivo*, or *in situ* assays, including, but not limited to those described herein.

**Germ-free:** An animal born and reared in aseptic conditions having no microorganisms living on or in it (for example, no bacteria in the gut of the animal).

**Gnotobiotic:** An animal in which only known strains of microorganisms are present. For example, a germ-free animal exposed to (*e.g*., intentionally inoculated with) one or more known bacterial strains is gnotobiotic. Germ-free animals are also gnotobiotic, as their microbial status is known. In contrast, conventionally reared animals (born and raised without absolute control of microorganism exposure) have a microbiota of many, and in most cases hundreds or thousands of organisms, which population will vary from animal to animal.

**Gut:** The term "gut" is used herein to refer to the digestive tract. Zebrafish do not have a stomach, rather their gut includes an intestinal bulb, the mid-intestine, and the caudal intestine (beginning at the esophageal junction and ending at the anus). The function of the zebrafish gut is analogous to the small and large intestine in mammals. Therefore, in some examples, gut refers to the intestine (such as the small and/or large intestine).

**Isolated:** An "isolated" or "purified" biological component (such as a nucleic acid, peptide, protein, or cell) has been substantially separated, produced apart from, or purified away from other biological components in which the component naturally occurs, for example, other chromosomal and extrachromosomal DNA and RNA, proteins, and/or cells. Nucleic acids, peptides and proteins that have been "isolated" or "purified" thus include nucleic acids and proteins purified by standard purification methods. The term also embraces nucleic acids, peptides and proteins prepared by recombinant expression in a host cell, as well as chemically synthesized nucleic acids or proteins.

The term "isolated" or "purified" does not require absolute purity; rather, it is intended as a relative term. Thus, for example, an isolated biological component is one in which the biological component is more enriched than the biological component is in its standard environment or a production vessel. Preferably, a preparation is purified such that the biological component represents at least 50%, such as at least 70%, at least 90%, at least 95%, or greater, of the total biological component content of the preparation.

**Heterologous:** Originating from a different genetic sources or species. For example, a nucleic acid that is heterologous to a cell originates from an organism or species other than the cell in which it is expressed. In one specific, non-limiting example, a heterologous nucleic acid includes an *Aeromonas veronii* nucleic acid that is present or expressed in a different bacterial cell (such as an *E. coli* cell) or in an algal, plant, or mammalian cell. Methods for introducing a heterologous nucleic acid into bacterial, algal, plant, and mammalian cells are well known in the art, for example transformation with a nucleic acid, including electroporation, lipofection, and particle gun acceleration.

In another example of use of the term heterologous, a nucleic acid operably linked to a heterologous promoter is from an organism or species other than that of the promoter. For example, an *Aeromonas veronii* acid may be linked to a heterologous bacterial, viral, or mammalian promoter. In other examples of the use of the term heterologous, a nucleic acid encoding a polypeptide (such as an anti-inflammatory polypeptide disclosed herein) or portion thereof is operably linked to a heterologous nucleic acid encoding a second polypeptide or portion thereof, for example to form a non-naturally occurring fusion protein.

**Inflammation:** A localized protective response elicited by injury to tissue that serves to sequester the inflammatory agent. Inflammation is orchestrated by a complex biological response of vascular tissues to harmful stimuli, such as pathogens, damaged cells, or irritants. It is a protective attempt by the organism to remove the injurious stimuli as well as initiate the healing process for the tissue. An inflammatory response is characterized by an accumulation of white blood cells, either systemically or locally at the site of inflammation. The inflammatory response may be measured by many methods well known in the art, such as the number of white blood cells, the number of polymorphonuclear leukocytes (PMN, such as neutrophils, eosinophils, basophils, and/or mast cells), a measure of the degree of PMN activation, or a measure of the amount of cytokines present.

A primary inflammation disorder is a disorder that is caused by the inflammation itself. A secondary inflammation disorder is inflammation that is the result of another disorder. Inflammation can lead to **inflammatory diseases,** such as rheumatoid arthritis, osteoarthritis, inflammatory lung disease (including chronic obstructive pulmonary lung disease), inflammatory bowel disease (including ulcerative colitis and Crohn's Disease), Hirschsprung disease (such as Hirschsprung associated enterocolitis), pelvic inflammatory disease, periodontal disease, polymyalgia rheumatica, atherosclerosis, systemic lupus erythematosus, systemic sclerosis, Sjogren's Syndrome, asthma, allergic rhinitis, and skin disorders (including dermatomyositis and psoriasis), irritable bowel syndrome, necrotizing enterocolitis, atopy, and the like.

Inflammation can be classified as either acute or chronic. Acute inflammation is the initial response of the body to harmful stimuli and is achieved by the increased movement of plasma and leukocytes from the blood into the injured tissues. A cascade of biochemical events propagates and matures the inflammatory response, involving the local vascular system, the immune system, and various cells within the injured tissue. Inflammation is typically self-limiting and resolution (for example, clearance of activated inflammatory cells) occurs when the threat of infection or tissue damage is eliminated. Prolonged inflammation, known as chronic inflammation, leads to a progressive shift in the type of cells which are present at the site of inflammation and is characterized by simultaneous destruction and healing of the tissue from the inflammatory process.

**Isolated:** An "isolated" biological component (such as a nucleic acid molecule, protein, or cell) has been substantially separated or purified away from other biological components in the cell of the organism, or the organism itself, in which the component naturally occurs, such as other chromosomal and extra-chromosomal DNA and RNA, proteins and/or cells. Nucleic acid molecules and proteins that have been "isolated" include nucleic acid molecules and proteins purified by standard purification methods or prepared by recombinant expression in a host cell, as well as chemically synthesized nucleic acid molecules and proteins.

**Myeloperoxidase (MPO):** An enzyme released by activated neutrophils that metabolizes hydrogen peroxide generated by the neutrophils and chloride ion to produce hypochlorous acid (HOCl), which is cytotoxic. MPO requires heme as a cofactor.

**Neutrophil:** A type of white blood cell (also known as neutrophil granulocytes) that is part of the class of polymorphonuclear leukocytes. They are the most abundant type of white blood cells in mammals. During acute inflammation, neutrophils are recruited to the site of inflammation or injury by chemotaxis toward chemokines, complement factors, leukotrienes, and/or fMLP. Neutrophils are phagocytic and can internalize and kill many microorganisms. They also release various cytotoxic compounds, including myeloperoxidase, defensins, cathepsin, alkaline phosphatase, lysozyme, NADPH oxidase, and gelatinase, through the process of degranulation.

**Operably linked:** A first nucleic acid is operably linked to a second nucleic acid when the first nucleic acid is placed in a functional relationship with the second nucleic acid. For instance, a promoter is operably linked to a coding sequence if the promoter affects the transcription or expression of the coding sequence. Generally, operably linked DNA sequences are contiguous and, where necessary to join two protein-coding regions, in the same reading frame.

**Recombinant:** A nucleic acid or protein that is not naturally occurring or has a sequence that is made by an artificial combination of two otherwise separated segments of nucleotides or amino acids. This artificial combination is often accomplished by chemical synthesis or, more commonly, by the artificial manipulation of isolated segments of nucleic acids, *e.g*., by genetic engineering techniques such as those described in Sambrook et al. Molecular Cloning: A Laboratory Manual, 3rd ed., Cold Spring Harbor Laboratory Press, NY, 2001. The term recombinant includes nucleic acids or proteins that have been altered solely by addition, substitution, or deletion of a portion of the nucleic acid sequence or amino acid sequence, respectively.

**Sample (or biological sample):** A specimen containing genomic DNA, RNA (including mRNA), protein, or combinations thereof, obtained from a subject. Examples include, but are not limited to, peripheral blood (or fractions thereof), fine needle aspirate, urine, saliva, feces, tissue biopsy, surgical specimen, and autopsy material. In one example, a sample includes a tumor biopsy (such as a colorectal tumor tissue biopsy) or an intestinal tissue biopsy.

**Sequence identity/similarity:** The identity/similarity between two or more nucleic acid sequences, or two or more amino acid sequences, is expressed in terms of the identity or similarity between the sequences. Sequence identity can be measured in terms of percentage identity; the higher the percentage, the more identical the sequences are. Sequence similarity can be measured in terms of percentage similarity (which takes into account conservative amino acid substitutions); the higher the percentage, the more similar the sequences are.

Methods of alignment of sequences for comparison are well known in the art. Various programs and alignment algorithms are described in: Smith & Waterman, Adv. Appl. Math. 2:482, 1981; Needleman & Wunsch, J. Mol. Biol. 48:443, 1970; Pearson & Lipman, Proc. Natl. Acad. Sci. USA 85:2444, 1988; Higgins & Sharp, Gene, 73:237-44, 1988; Higgins & Sharp, CABIOS 5:151-3, 1989; Corpet et al., Nuc. Acids Res. 16:10881-90, 1988; Huang et al., Computer Appls. in the Biosciences 8, 155-65, 1992; and Pearson et al., Meth. Mol. Bio. 24:307-31, 1994. Altschul et al., J. Mol. Biol. 215:403-10, 1990, presents a detailed consideration of sequence alignment methods and homology calculations. The NCBI Basic Local Alignment Search Tool (BLAST) (Altschul et al., J. Mol. Biol. 215:403-10, 1990) is available from several sources, including the National Center for Biotechnology (NCBI, National Library of Medicine, Building 38A, Room 8N805, Bethesda, MD 20894) and on the Internet, for use in connection with the sequence analysis programs blastp, blastn, blastx, tblastn and tblastx. Additional information can be found at the NCBI web site.

One of skill in the art will appreciate that the particular sequence identity ranges provided herein are for guidance only; it is possible that strongly significant homologs or orthologs could be obtained that fall outside the ranges provided.

**Subject:** Living multi-cellular vertebrate organism, a category that includes vertebrates, including human and non-human mammals.

**Therapeutically effective amount:** An amount of an agent or composition that alone, or together with a pharmaceutically acceptable carrier and/or one or more additional therapeutic agents, induces the desired response. Effective amounts of an agent can be determined in many different ways, such as assaying for a reduction in inflammation, delay (or even prevention) of onset of a condition associated with inflammation (such as inflammatory bowel disease), or a reduction or amelioration of one or more symptoms of a subject with inflammation. Effective amounts also can be determined through various *in vitro*, *in vivo*, or *in situ* assays.

**Transduced** and **Transformed:** A virus or vector "transduces" a cell when it transfers nucleic acid into the cell. A cell is "transformed" by a nucleic acid transduced into the cell when the DNA becomes replicated by the cell, either by incorporation of the nucleic acid into the cellular genome, or by episomal replication. As used herein, the term transformation encompasses all techniques by which a nucleic acid molecule might be introduced into such a cell, including transfection with viral vectors, transformation with plasmid vectors, and introduction of naked DNA by electroporation, lipofection, or particle gun acceleration.

**Treating or Inhibiting:** "Inhibiting" refers to inhibiting or reducing the full development of a condition or symptom (such as inflammation or an inflammatory response) or a disorder (such as an inflammatory disease). Inhibition of a condition or disease can span the spectrum from partial inhibition (reduction) to substantially complete inhibition (prevention) of the condition, symptom, or disease. In some examples, the term "inhibiting" refers to reducing or delaying the onset or progression of inflammation, an inflammatory response, or an inflammatory disease. In contrast, "treatment" refers to a therapeutic intervention that ameliorates a sign or symptom of a disease or pathological condition (such as an inflammatory disease) after it has begun to develop.

**Vector:** A nucleic acid molecule that can be introduced into a host cell, thereby producing a transformed or transduced host cell. Recombinant DNA vectors are vectors including recombinant DNA. A vector can include nucleic acid sequences that permit it to replicate in a host cell, such as an origin of replication. A vector can also include one or more selectable marker genes, a cloning site for introduction of heterologous nucleic acids, a promoter (for example for expression of an operably linked nucleic acid), and/or other genetic elements known in the art. Vectors include plasmid vectors, including plasmids for expression in gram negative and gram positive bacterial cell. Exemplary vectors include those for use in *E. coli.* Vectors also include viral vectors, such as, but not limited to, retrovirus, orthopox, avipox, fowlpox, capripox, suipox, adenovirus, herpes virus, alpha virus, baculovirus, Sindbis virus, vaccinia virus, and poliovirus vectors. Vectors also include vectors for expression in yeast cells.

In some examples, a heterologous nucleic acid (such as a nucleic acid encoding an *A*. *veronii* protein) is introduced into a vector to produce a recombinant vector, thereby allowing the nucleic acid to be renewably produced and or a protein encoded by the nucleic acid to be expressed.

### III. Anti-Inflammatory Protein from Aeromonas

Disclosed herein are anti-inflammatory proteins from members of the intestinal microbiota, including *Aeromonas veronii.* In some embodiments, the anti-inflammatory protein is a polypeptide for use in a method as defined in claim 1, the sequence of which comprises or consists of the amino acid sequence as set forth as:

In additional embodiments, an anti-inflammatory polypeptide disclosed herein has at least 85%, 90%, 95%, 96%, 97%, 98%, or 99% sequence identity to the amino acid sequence set forth in SEQ ID NO: 1, for use in a method as defined in claim 1. As disclosed herein, the anti-inflammatory protein may not include the signal sequence, which is removed from the mature protein. In some examples, the signal sequence is predicted to include the first 22 amino acids of SEQ ID NO: 1. Thus, in some examples, the mature polypeptide can have an amino acid sequence with at least 80%, 85%, 90%, 95%, 96%, 97%, 98%, 99%, or 100% sequence identity to amino acid sequences 23-313 of SEQ ID NO: 1. Exemplary sequences can be obtained using computer programs that are readily available on the internet and the amino acid sequences set forth herein. In some examples, the polypeptide retains a function of the anti-inflammatory protein, such as decreasing the number or activation of neutrophils and/or reducing or inhibiting inflammation in a subject.

As disclosed herein, an anti-inflammatory protein (such as *A. veronii* AP) may include a portion or fragment of the protein (for example, a portion of an *A. veronii* AP disclosed herein). In some examples, the anti-inflammatory protein or portion thereof includes at least 20 contiguous amino acids of AP, for example, at least 30, at least 50, at least 75, at least 100, at least 150, at least 200, at least 250, at least 300, at least 350, at least 400, at least 450, or more amino acids of AP. In one example, a fragment of AP includes the mature protein (for example, AP that does not include the signal sequence, such as amino acids 23-313 of SEQ ID NO: 1). In other examples, a portion or fragment of an anti-inflammatory protein includes one or more domains of AP. In some examples, a domain may include a portion of AP with structural similarity to a β-trefoil domain (for example, with similarity to a BTD found in one or more cytokines), such as amino acids 103-176 of SEQ ID NO: 1 or amino acids 148-176 of SEQ ID NO: 1. One of ordinary skill in the art will recognize that the boundaries of the domain are not exact and in some examples may include additional or fewer amino acids (for example, about 20, 19, 18, 17, 16, 15, 14, 13, 12, 11, 10, 9, 8, 7, 6, 5, 4, 3, 2, or 1 more or less amino acids from either end of the domain). Furthermore, one of ordinary skill in the art can identify corresponding anti-inflammatory AP proteins and/or domains from other anti-inflammatory AP proteins, for example a corresponding AP from a bacterium other than *A. veronii* or from another organism. Exemplary proteins related to the *A. veronii* AP protein (such as SEQ ID NO: 1) include proteins from *Aeromonas salmonicida* (*e.g*., GenBank Accession No. WP_034524138.1), *Aeromonas sobria* (*e.g*., GenBank Accession No.WP_042019195.1), *Aeromonas bestiarum* (*e.g*., GenBank Accession No. WP_043556138), *Aeromonas piscicola* (*e.g*., GenBank Accession No. WP_042869769.1), and *Aeromonas jandaei* (*e.g*., GenBank Accession No. WP_041209781.1).

Minor modifications of an anti-inflammatory protein primary amino acid sequence (such as the *Aeromonas* AP disclosed herein) are also disclosed herein. Such modifications may result in polypeptides that have substantially equivalent activity as compared to the unmodified counterpart polypeptide described herein. Such modifications may be deliberate, for example as by site-directed mutagenesis, or may be spontaneous. All of the polypeptides produced by these modifications are included herein. Thus, a specific, non-limiting example of an anti-inflammatory protein is a conservative variant of the protein (such as a single conservative amino acid substitution, for example, one or more conservative amino acid substitutions, for example 1-10 conservative substitutions, 2-5 conservative substitutions, 4-9 conservative substitutions, such as 1, 2, 5 or 10 conservative substitutions). In other examples, the protein may include one or more non-conservative substitutions (for example 1-10 non-conservative substitutions, 2-5 non-conservative substitutions, 4-9 non-conservative substitutions, such as 1, 2, 5 or 10 non-conservative substitutions), so long as the protein retains anti-inflammatory activity.

In additional embodiments, the anti-inflammatory polypeptide for use in a method as defined in claim 1 includes a tag (such as an N-terminal or C-terminal tag), for example for use in protein purification. One of skill in the art can select appropriate tags, such as a His-tag, a GST tag, or an antibody recognition sequence (such as a Myc-tag or HA-tag). The anti-inflammatory polypeptide can also be produced as a fusion protein, either to facilitate expression and/or purification or to facilitate delivery to a subject. For example, fusion proteins including a therapeutic molecule (such as the disclosed anti-inflammatory proteins) and transferrin has been shown to be useful for oral delivery routes. In other examples, the disclosed anti-inflammatory polypeptides may include a detectable label, such as a radioisotope, fluorophore, or hapten.

Additional exemplary *Aeromonas* anti-inflammatory proteins include the amino acid sequences of GenBank Accession Nos. WP_021230730 (SEQ ID NO: 1), WP_005340784, WP_005357002, YP_004390739, WP_005342535, YP_001142339, WP_021140301, and WP_005310485; as present in GenBank on May 16, 2014. One of ordinary skill in the art can identify additional candidate anti-inflammatory proteins related to the anti-inflammatory proteins disclosed herein, for example from other microbiota (for example, other bacteria from the zebrafish gut or bacteria from mammalian gut, such as human microbiota).

The structure of SEQ ID NO: 1 (also referred to herein as AP or protein 1882) was analyzed for conserved features using HHpred (available on the World Wide Web at toolkit.tuebingen.mpg.de/hhpred) using the AP sequence and selecting all protein databases. As shown in FIG. 7A, the N-terminus showed similarity to secretion signal sequences, consistent with this protein being processed by the type II secretion system (discussed in Example 1). In addition, the protein showed similarity to decaheme cytochrome C of *Shewanella oneidensis* (amino acids 6-175 of SEQ ID NO: 1) and to a beta-trefoil domain (BTD) that is also found in cytokines, including interleukin-18 (IL-18), and interleukin-lb (IL-lb). In some examples, the BTD domain includes amino acids 103-176 of SEQ ID NO: 1 or amino acids 148-176 of SEQ ID NO: 1.

As discussed above, AP includes a region that is predicted to contain a β-trefoil domain with homology to human cytokines IL-1β, IL-1α, and IL-1ra. Both IL-1β and IL-1α bind the type 1 IL-1 receptor (IL-1R1). IL-1R1 engages with the IL-1 receptor accessory protein (IL-1RAP) to form a complex that results in the recruitment of the MyD88 adaptor protein (FIG. 7B). This initiates a signaling cascade that causes the transcription factor, NF-κB, to translocate into the nucleus and initiate transcription of the pro-inflammatory cytokines *Il1*β*, Il6, Il8*, and *tnf*α*.* Interleukin 1 receptor antagonist (IL-1ra) dampens IL-1 signaling by binding IL-1R1 and preventing receptor complex formation with IL-1RAP. Normal function and levels of IL-1ra affect ulcerative colitis, and polymorphisms in the IL-1ra gene are associated with severity and susceptibility to UC (Carter et al., Genes Immun. 5:8-15, 2004), neutralizing IL-1ra exacerbates colitis (Feretti et al., J. Clin. Invest. 94:449-453, 1994), and administering exogenous IL-1ra (Ricci et al., BMC Biotechnol. 3:15, 2003) or inducing endogenous IL-1ra (Gresnigt et al., PLoS Pathog. 10:e1003936, 2014) reduces disease severity in animal models of inflammation (Dinarello, Blood 89:2095-2147, 1996; Dinarello Blood 118:3720-3732, 2011). The IL-1 family of cytokines (Huising et al. Dev. Comp. Immunol. 28:395-413, 2004) and neutrophil behavior and activity (Renshaw et al., Blood 108:3976-3978, 2006; Guyader et al. Blood 111:132-141, 2008) are conserved in zebrafish. Moreover, intestinal neutrophil influx in zebrafish depends on Myd88 (Bates et al., Cell Host Microbe 2:371-382, 2007), confirming this pathway's role in neutrophil behavior. Without being bound by theory, based on the biological activity and homology of AP, it is believed that AP may be a competitive inhibitor of binding sites on the cytokine receptor IL-1R1, potentially decreasing pro-inflammatory IL-1 signaling.

In additional embodiments, the anti-inflammatory polypeptide disclosed herein is for use in a method as defined in claim 1, and encoded by a nucleic acid sequence which comprises or consists of the nucleic acid sequence set forth as:

In additional embodiments, a nucleic acid encoding an *Aeromonas* anti-inflammatory polypeptide disclosed herein is for use in a method as defined in claim 1, and has at least 85%, 90%, 95%, 96%, 97%, 98%, or 99% sequence identity to the nucleic acid sequence set forth in SEQ ID NO: 2 or a fragment thereof. For example, the nucleic acid can have a nucleic acid sequence with at least 80%, 85%, 90%, 95%, 96%, 97%, 98%, 99%, or 100% sequence identity to a nucleic acid encoding amino acids 23-313 of SEQ ID NO: 1 (such as nucleotides 67-942 of SEQ ID NO: 2) or a nucleic acid encoding amino acids 103-176 of SEQ ID NO: 1 (such as nucleotides 307-528 of SEQ ID NO: 2). Exemplary sequences can be obtained using computer programs that are readily available on the internet and the amino acid sequences set forth herein. In some examples, the nucleic acid encodes a polypeptide that retains a function of the anti-inflammatory protein, such as decreasing the number or activation of neutrophils and/or reducing or inhibiting inflammation in a subject.

Additional exemplary *Aeromonas* nucleic acids encoding the disclosed proteins include the nucleic acid sequences of GenBank Accession Nos. NZ_ATFB01000030 (nucleotides 64518-65459; SEQ ID NO: 2), NZ_JH815583 (nucleotides 116798-117739, complement), NZ_JH815589 (nucleotides 391175-392116, complement), NC_015424 (nucleotides 103273-104214), NZ_JH823256 (nucleotides 1061308-1062153), NC_009348 (nucleotides 2743728-2744681, complement), NZ_ARYZ01000054 (nucleotides 2343-3290), and NZ_AGVO01000002 (nucleotides 295610-297457); as present in GenBank on May 16, 2014. One of ordinary skill in the art can identify additional candidate nucleic acids encoding anti-inflammatory proteins related to the anti-inflammatory proteins disclosed herein, for example from other microbiota.

Minor modifications of nucleic acids encoding an anti-inflammatory protein primary amino acid sequence (such as the *Aeromonas* AP disclosed herein) are also contemplated herein. Such modifications to the nucleic acid may result in polypeptides that have substantially equivalent activity as compared to the unmodified counterpart polypeptide described herein. Such modifications may be deliberate, for example as by site-directed mutagenesis, or may be spontaneous. All of the nucleic acids produced by these modifications are included herein. Thus, a specific, non-limiting example of modified nucleic acid encoding an anti-inflammatory protein is a nucleic acid encoding conservative variant of the protein (such as a single conservative amino acid substitution, for example, one or more conservative amino acid substitutions, for example 1-10 conservative substitutions, 2-5 conservative substitutions, 4-9 conservative substitutions, such as 1, 2, 5 or 10 conservative substitutions). In other examples, the nucleic acid may encode a protein including one or more non-conservative substitutions (for example 1-10 non-conservative substitutions, 2-5 non-conservative substitutions, 4-9 non-conservative substitutions, such as 1, 2, 5 or 10 non-conservative substitutions), so long as the encoded protein retains anti-inflammatory activity.

In additional embodiments, the nucleic acid encoding the anti-inflammatory polypeptide for use in a method as defined in claim 1 further includes a nucleic acid sequence encoding a tag (such as an N-terminal or C-terminal tag), for example for use in protein purification. One of skill in the art can select nucleic acids encoding appropriate tags, such as a His-tag, a GST tag, or an antibody recognition sequence (such as a Myc-tag or HA-tag). The nucleic acid may also encode a fusion, for example, a nucleic acid encoding a fusion protein including a disclosed anti-inflammatory proteins and transferrin. In other examples, the disclosed nucleic acids may include a detectable label, such as a radioisotope, fluorophore, or hapten.

Nucleic acid molecules encoding an anti-inflammatory protein disclosed herein also include a recombinant DNA which is incorporated into a vector, into an autonomously replicating plasmid or virus, or into the genomic DNA of a prokaryote or eukaryote, or which exists as a separate molecule (such as a cDNA) independent of other sequences. A nucleic acid encoding an anti-inflammatory polypeptide (such as an *Aeromonas* anti-inflammatory peptide, for example SEQ ID NO: 1 or a fragment thereof) is in some examples operably linked to heterologous expression control sequences. An expression control sequence operably linked to a coding sequence is ligated such that expression of the coding sequence is achieved under conditions compatible with the expression control sequences. The expression control sequences include, but are not limited to, appropriate promoters, enhancers, transcription terminators, a start codon (*e.g*., ATG) in front of a protein-encoding nucleic acid, splicing signal for introns, maintenance of the correct reading frame of that gene to permit proper translation of mRNA, and stop codons. The expression control sequence(s) in some examples are heterologous expression control sequence(s), for example from an organism or species other than the protein-encoding nucleic acid. Thus, the protein-encoding nucleic acid operably linked to a heterologous expression control sequence (such as a promoter) comprises a nucleic acid that is not naturally occurring. In other examples, the nucleic acid is operably linked to a tag sequence (such as 6xHis, HA tag, or Myc tag) or another protein-coding sequence, such as glutathione S-transferase or maltose binding protein.

Vectors for cloning and replication of the disclosed nucleic acid molecules include bacterial plasmids, such as bacterial cloning or expression plasmids. Exemplary bacterial plasmids into which the nucleic acids can be cloned include *E. coli* plasmids, such as pBR322, pUC plasmids (such as pUC18 or pUC19), pBluescript, pACYC184, pCD1, pGEM® plasmids (such as pGEM®-3, pGEM®-4, pGEM-T® plasmids; Pomega, Madison, WI), TA-cloning vectors, such as pCR® plasmids (for example, pCR® II, pCR® 2.1, or pCR® 4 plasmids; Life Technologies, Grand Island, NY) or pcDNA plasmids (for example pcDNA™3.1 or pcDNA™3.3 plasmids; Life Technologies). In some examples, the vector includes a heterologous promoter which allows protein expression in bacteria. Exemplary vectors include pET vectors (for example, pET-21b), pDEST™ vectors (Life Technologies), pRSET vectors (Life Technologies), pBAD vectors, and pQE vectors (Qiagen). The disclosed nucleic acids can be also be cloned into *B. subtilis* plasmids, for example, pTA1060 and pHT plasmids (such as pHT01, pHT43, or pHT315 plasmids). One of skill in the art can select additional vectors suitable for cloning and/or bacterial expression of anti-inflammatory proteins such as those disclosed herein.

In other embodiments, vectors are used for expression in yeast such as *S. cerevisiae* or *Kluyveromyces lactis.* Several promoters are known to be of use in yeast expression systems such as the constitutive promoters plasma membrane H⁺-ATPase (*PMA1*), glyceraldehyde-3-phosphate dehydrogenase (*GPD*), phosphoglycerate kinase-1 (*PGK1*), alcohol dehydrogenase-1 *(ADH1),* and pleiotropic drug-resistant pump (*PDR5*). In addition, many inducible promoters are of use, such as *GAL1-10* (induced by galactose), *PHO5* (induced by low extracellular inorganic phosphate), and tandem heat shock *HSE* elements (induced by temperature elevation to 37°C). Promoters that direct variable expression in response to a titratable inducer include the methionine-responsive *MET3* and *MET25* promoters and copper-dependent *CUP1* promoters. Any of these promoters may be cloned into multicopy (2µ) or single copy (*CEN*) plasmids to give an additional level of control in expression level. The plasmids can include nutritional markers (such as *URA3*, *ADE3*, *HIS1*, and others) for selection in yeast and antibiotic resistance (such as *AMP*) for propagation in bacteria. Plasmids for expression on *K. lactis* are known, such as pKLAC1. Thus, in one example, after amplification in bacteria, plasmids can be introduced into the corresponding yeast auxotrophs by methods similar to bacterial transformation.

Viral vectors including the disclosed polynucleotides (such as polynucleotides encoding an anti-inflammatory protein) can also be prepared. A number of viral vectors have been constructed, including polyoma, SV40 (Madzak et al., 1992, J. Gen. Virol., 73:15331536), adenovirus (Berkner, 1992, Curr. Top. Microbiol. Immunol., 158:39-6; Berliner et al., 1988, BioTechniques, 6:616-629; Gorziglia et al., 1992, J. Virol., 66:4407-4412; Quantin et al., 1992, Proc. Natl. Acad. Sci. USA, 89:2581-2584; Rosenfeld et al., 1992, Cell, 68:143-155; Wilkinson et al., 1992, Nucl. Acids Res., 20:2233-2239; Stratford-Perricaudet et al., 1990, Hum. Gene Ther., 1:241-256), vaccinia virus (Mackett et al., 1992, Biotechnology, 24:495-499), adeno-associated virus (Muzyczka, 1992, Curr. Top. Microbiol. Immunol. 158:91-123; On et al., 1990, Gene, 89:279-282), herpes viruses including HSV and EBV (Margolskee, 1992, Curr. Top. Microbiol. Immunol., 158:67-90; Johnson et al., 1992, J. Virol., 66:2952-2965; Fink et al., 1992, Hum. Gene Ther. 3:11-19; Breakfield et al., 1987, Mol. Neurobiol., 1:337-371; Fresse et al., 1990, Biochem. Pharmacol., 40:2189-2199), Sindbis viruses (Herweijer et al., 1995, Hum. Gene Ther. 6:1161-1167; U.S. Patent Nos. 5,091,309 and 5,2217,879), alphaviruses (S. Schlesinger, 1993, Trends Biotechnol. 11:18-22; Frolov et al., 1996, Proc. Natl. Acad. Sci. USA 93:11371-11377) and retroviruses of avian (Brandyopadhyay et al., 1984, Mol. Cell Biol., 4:749-754; Petropouplos et al., 1992, J. Virol., 66:3391-3397), murine (Miller, 1992, Curr. Top. Microbiol. Immunol., 158:1-24; Miller et al., 1985, Mol. Cell Biol., 5:431-437; Sorge et al., 1984, Mol. Cell Biol., 4:1730-1737; Mann et al., 1985, J. Virol., 54:401-407), and human origin (Page et al., 1990, J. Virol., 64:5370-5276; Buchschalcher et al., 1992, J. Virol., 66:2731-2739). Baculovirus (Autographa californica multinuclear polyhedrosis virus; AcMNPV) vectors are also known in the art, and may be obtained from commercial sources (such as PharMingen, San Diego, Calif.; Protein Sciences Corp., Meriden, Conn.; Stratagene, La Jolla, Calif.).

DNA sequences encoding an anti-inflammatory polypeptide can be expressed *in vitro* by DNA transfer into a suitable host cell. The cell may be prokaryotic or eukaryotic. The term also includes any progeny of the subject host cell. It is understood that all progeny may not be identical to the parental cell since there may be mutations that occur during replication. Methods of stable transfer, meaning that the foreign DNA is continuously maintained in the host, are known in the art.

Host cells can include microbial, yeast, insect and/or mammalian host cells. Methods of expressing DNA sequences having eukaryotic or viral sequences in prokaryotes are well known in the art. Non-limiting examples of suitable host cells include bacteria, archea, insect, fungi (for example, yeast), mycobacterium (such as *M. smegmatis*), plant, and animal cells (for example, mammalian cells, such as human cells). Exemplary cells of use include *E. coli, Bacillus subtilis, Saccharomyces cerevisiae, Salmonella typhimurium,* SF9 cells, C129 cells, 293 cells, *Neurospora*, and immortalized mammalian myeloid and lymphoid cell lines. Techniques for the propagation of mammalian cells in culture are well-known (see, Jakoby and Pastan (eds), 1979, Cell Culture. Meth. Enzymol., volume 58, Academic Press, Inc., Harcourt Brace Jovanovich, N.Y.). Examples of commonly used mammalian host cell lines are VERO and HeLa cells, CHO cells, and WI38, BHK, and COS cell lines, although other cell lines may be used, such as cells designed to provide higher expression, desirable glycosylation patterns, or other features. As discussed above, techniques for the transformation of yeast cells, such as polyethylene glycol transformation, protoplast transformation and gene guns are also known in the art (see Gietz and Woods Meth. Enzymol. 350: 87-96, 2002).

Transformation of a host cell with recombinant DNA can be carried out by conventional techniques as are well known to those skilled in the art. Where the host is prokaryotic, such as, but not limited to, *E. coli,* competent cells which are capable of DNA uptake can be prepared from cells harvested after exponential growth phase and subsequently treated by the CaCl₂ method using procedures well known in the art. Alternatively, MgCl₂ or RbCl can be used. Transformation can also be performed after forming a protoplast of the host cell if desired, or by electroporation.

When the host is a eukaryote, such methods of transfection of DNA as calcium phosphate coprecipitates, conventional mechanical procedures such as microinjection, electroporation, insertion of a plasmid encased in liposomes, or virus vectors can be used. Eukaryotic cells can also be co-transformed with a polynucleotide encoding an anti-inflammatory protein polypeptide and a second foreign DNA molecule encoding a selectable phenotype, such as the herpes simplex thymidine kinase gene. Another method is to use a eukaryotic viral vector, such as simian virus 40 (SV40) or bovine papilloma virus, to transiently infect or transform eukaryotic cells and express the protein (see for example, Eukaryotic Viral Vectors, Cold Spring Harbor Laboratory, Gluzman ed., 1982).

### IV. Methods of Treating or Inhibiting Inflammation

Disclosed herein are methods of treating or inhibiting inflammation in a subject. In some embodiments, the methods include administering to a subject an effective amount of a microbial anti-inflammatory protein (such as an *Aeromonas* anti-inflammatory protein), including, but not limited to the anti-inflammatory proteins disclosed herein. As disclosed herein, the anti-inflammatory protein may be administered in any form, including administration of cells producing an anti-inflammatory protein disclosed herein (*e.g., A. veronii, A. salmonicida*, or *A. hydrophila*, or other bacteria recombinantly expressing or overexpressing an anti-inflammatory protein), a cell extract, or a preparation (such as a cell-free supernatant) from a cell producing an anti-inflammatory protein, an isolated or purified anti-inflammatory protein (including, but not limited to SEQ ID NO: 1 or a fragment thereof), or a nucleic acid encoding an anti-inflammatory protein (including, but not limited to, SEQ ID NO: 2).

The anti-inflammatory proteins disclosed herein can be chemically synthesized by standard methods, or can be produced recombinantly. An exemplary process for polypeptide production is described in Lu et al., FEBS Lett. 429:31-35, 1998. They can also be isolated by methods including preparative chromatography and immunological separations. Polypeptides can also be produced using molecular genetic techniques, such as by inserting a nucleic acid encoding an anti-inflammatory protein or a portion thereof into an expression vector, introducing the expression vector into a host cell (such as *E. coli*), and isolating the polypeptide (for example, as discussed in Section III). In some examples, the protein includes a tag (such as an N-terminal or C-terminal tag), for example for use in protein purification. One of skill in the art can select appropriate tags, such as a His-tag, a GST tag, or an antibody recognition sequence (such as a Myc-tag or HA-tag). In some embodiments, the anti-inflammatory polypeptide for use in a method as defined in claim 1 is produced by bacteria (such as *Aeromonas* or another suitable bacteria) expressing the anti-inflammatory protein from an expression vector (such as a vector including a constitutive or a regulatable promoter). As disclosed herein, the anti-inflammatory polypeptide may be administered to a subject as an isolated preparation from the bacteria, an extract or other preparation (such as cell-free supernatant), or the recombinant bacteria may be administered to the subject.

In some embodiments, the anti-inflammatory polypeptide for use in a method as defined in claim 1 (such as a polypeptide having the sequence of SEQ ID NO: 1 or a polypeptide that is at least 80%, 85%, 90%, 95%, 98%, or 99% identical to SEQ ID NO: 1 or a fragment thereof) is administered to a subject to treat or inhibit inflammation. In some examples, a fragment of the anti-inflammatory polypepide includes the processed mature protein, for example, lacking the signal sequence (such as amino acids 23-313 of SEQ ID NO: 1) or a domain of the protein (such as a BTD domain, for example amino acids 103-176 of SEQ ID NO: 1 or amino acids 148-176 of SEQ ID NO: 1), or polypeptides that are at least 80%, 85%, 90%, 95%, 98%, or 99% identical to the fragment of the anti-inflammatory protein. In some examples, administration of the anti-inflammatory polypeptide or fragment thereof reduces at least one marker of inflammation (such as number or activation of neutrophils) by at least about 10% (such as at least 15%, at least 20%, at least 25%, at least 30%, at least 40%, at least 50%, at least 60%, at least 70%, at least 80%, at least 90%, or more).

In some examples, the subject may have one of a number of conditions broadly categorized as an inflammatory disease or disorder. Such disorders include, but are not limited to, rheumatoid arthritis, osteoarthritis, inflammatory lung disease (including chronic obstructive pulmonary lung disease), inflammatory bowel disease (including ulcerative colitis and Crohn's Disease), Hirschsprung disease (such as Hirschsprung's associated enterocolitis), pelvic inflammatory disease, periodontal disease, polymyalgia rheumatica, atherosclerosis, systemic lupus erythematosus, systemic sclerosis, Sjogren's Syndrome, asthma, allergic rhinitis, and skin disorders (including dermatomyositis and psoriasis), irritable bowel syndrome, necrotizing enterocolitis, or atopy. In particular embodiments, the subject has inflammatory bowel disease.

The anti-inflammatory protein, nucleic acid encoding the anti-inflammatory protein, or cell expressing the anti-inflammatory protein can be administered to a subject in need of treatment using any suitable means known in the art. Methods of administration include, but are not limited to, intradermal, intramuscular, intraperitoneal, parenteral, subcutaneous, rectal, intranasal, inhalation, oral, or by gene gun. Intranasal administration refers to delivery of the compositions into the nose and nasal passages through one or both of the nares and can include delivery by a spraying mechanism or droplet mechanism, or through aerosolization of the therapeutic agent. In particular examples, the anti-inflammatory protein, nucleic acid encoding the anti-inflammatory protein, or a preparation including the anti-inflammatory protein (such as a cell extract or preparation or cells expressing the protein) is administered orally. In other examples, the anti-inflammatory protein, nucleic acid encoding the anti-inflammatory protein, or a preparation encoding the anti-inflammatory protein (such as a cell extract or preparation or cells expressing the protein) is administered subcutaneously or intramuscularly.

Therapeutic agents can be administered in any suitable manner, preferably with pharmaceutically acceptable carriers. Pharmaceutically acceptable carriers are determined in part by the particular composition being administered, as well as by the particular method used to administer the composition. Accordingly, there is a wide variety of suitable formulations of pharmaceutical compositions of the present disclosure. The pharmaceutically acceptable carriers (vehicles) useful in this disclosure are conventional. Remington: The Science and Practice of Pharmacy, The University of the Sciences in Philadelphia, Editor, Lippincott, Williams, & Wilkins, Philadelphia, PA, 21st Edition (2005), describes compositions and formulations suitable for pharmaceutical delivery of one or more therapeutic agents

Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

Compositions for oral administration include powders or granules, suspensions or solutions in water or non-aqueous media, capsules, sachets, or tablets. Thickeners, flavorings, diluents, emulsifiers, dispersing aids or binders may be desirable.

The amount of anti-inflammatory protein, nucleic acid encoding the anti-inflammatory protein, or a preparation encoding the anti-inflammatory protein (such as a cell extract or preparation or cells expressing the protein) to be administered to a subject can be selected by one of ordinary skill in the art, for example from about 1 µg to 5 g anti-inflammatory protein (such as about 10 µg to 1 g, 100 µg to 500 mg, or 1 mg to 100 mg). In other examples, the amount of anti-inflammatory protein or a preparation encoding the anti-inflammatory protein (such as a cell extract or preparation or cells expressing the protein) to be administered to a subject is about 0.001 mg/kg to about 1000 mg/kg (such as about 0.01 mg/kg to about 500 mg/kg, about 1 mg/kg to about 250 mg/kg, or about 10 mg/kg to 100 mg/kg).

The dosage can be administered one or more times per day, in divided doses (such as 2, 3, or 4 divided doses per day), or in a single dosage daily. The dosage can also be administered every 2 days, every 3 days, bi-weekly, once weekly, semi-weekly, or monthly. In some examples, an effective amount of anti-inflammatory protein is an amount that inhibits or ameliorates one or more symptoms of an inflammatory disease. In other examples, an effective amount of anti-inflammatory protein is an amount that decreases one or more markers of inflammation, such as number of white blood cells (such as neutrophils).

In particular examples, prior to, during, or following administration of a disclosed anti-inflammatory protein (or nucleic acid encoding the protein, or preparation of bacteria expressing the protein) the subject can receive one or more other anti-inflammatory therapies. Examples of such therapies include, but are not limited to, non-steroidal anti-inflammatory drugs (NSAIDs, such as aspirin, ibuprofen, naproxen, and celecoxib), corticosteroids (such as prednisone, methylprednisolone, or cortisone), or DMARDs (such as etanercept, adalimumab, infliximab, rituximab, or methotrexate). Combinations of these therapies can also be administered to a subject.

### V. Methods of Identifying Modulators of Inflammation

Disclosed herein are methods for identifying modulators of immune responses, such as inflammation. As disclosed herein, methods include inoculating germ-free zebrafish (or a population of germ-free zebrafish) with one or more defined bacterial strains or CFS supernatant from one or more defined bacterial strains and/or one or more test compounds and determining the amount of inflammation, for example by measuring a marker of inflammation. In some examples, the zebrafish are transgenic for one or more genes, for example, are transgenic for green fluorescent protein (GFP) expressed under the control of the myeloperoxidase (MPO) promoter. As disclosed herein, the methods include inoculating conventionally raised zebrafish (or a population of conventionally raised zebrafish) with one or more test compounds and determining the amount of inflammation, for example by measuring a marker of inflammation. In some examples, the conventionally raised zebrafish are transgenic or mutant for one or more genes, for example, *Sox10.*

In some examples, a marker of inflammation is the amount (such as number or percentage) of neutrophils or macrophages present, for example in the gut of the zebrafish. Presence or amount of neutrophils or macrophages in the gut can be determined by histological staining, *in situ* hybridization, or immunohistochemistry. In one example, presence or amount of neutrophils is determined by detection of a marker expressed under the control of a neutrophil-specific gene (such as green fluorescent protein (GFP) expressed from the myeloperoxidase (MPO) promoter, as described below). In other examples, a marker of inflammation is expression (such as the amount of expression) of one or more inflammatory genes, for example cytokines (such as TNFα) or expression of MPO. Expression of markers of inflammation can be detected using methods such as PCR (for example, RT-PCR, real-time PCR, quantitative real-time RT-PCR), *in situ* hybridization, Northern blotting, immunohistochemistry, Western blotting, flow cytometry, microscopy, or other techniques known to one of skill in the art.

In some examples, the presence or amount of the marker of inflammation in a zebrafish contacted or treated with a test compound is compared to a control. In some examples, the control is a zebrafish (or population of zebrafish) treated under the same conditions, but without treatment with the test compound. A decrease in the presence or amount of the marker of inflammation in the treated zebrafish (such as an decrease of at least about 10%, about 20%, about 50%, about 80%, about 90%, about 1.5-fold, about 2-fold, about 3-fold, about 5-fold, about 10-fold or more) as compared to in the control indicates that the compound inhibits immune response or inflammation.

Bacterial strains or test compounds identified as an inhibitor of immune response or inflammation may be selected for further testing. If the inhibitor is a bacterial strain or CFS from a bacterial strain, additional testing may be carried out to identify or purify one or more anti-inflammatory compounds from the bacterial strain.

Bacterial strains that may be used in the screening methods disclosed herein (either for inoculation of germ-free zebrafish or for preparing CFS with which the zebrafish are contacted) include, but are not limited to *Aeromonas*, *Vibrio, Variovorax, Delftia, Acinetobacter, Shewanella, Chitinibacter, Bosea, Exiguobacterium, Carnobacterium, Ensifer, Korcuia, Comamonas,* and *Lactobacillus.* Additional bacterial strains, such as additional strains found in the zebrafish or mammalian gut (such as the human gut) can also be tested for anti-inflammatory activity in the methods disclosed herein.

A "compound" or "test compound" is any substance or any combination of substances that is useful for achieving an end or result. Any compound that has potential (whether or not ultimately realized) to modulate immune response or inflammation can be tested using the methods of this disclosure.

Exemplary compounds include, but are not limited to, peptides, such as soluble peptides, including but not limited to members of random peptide libraries (see, *e.g*., Lam et al., Nature, 354:82-84, 1991; Houghten et al., Nature, 354:84-86, 1991), and combinatorial chemistry-derived molecular libraries made of D-and/or L-configuration amino acids, phosphopeptides (including, but not limited to, members of random or partially degenerate, directed phosphopeptide libraries; see, *e.g*., Songyang et al., Cell, 72:767-778, 1993), antibodies (including, but not limited to, polyclonal, monoclonal, humanized, anti-idiotypic, chimeric or single chain antibodies, and Fab, F(ab')₂ and Fab expression library fragments, and epitope-binding fragments thereof), small organic or inorganic molecules (such as, so-called natural products or members of chemical combinatorial libraries), molecular complexes (such as protein complexes), or nucleic acids (such as antisense compounds).

Appropriate compounds can be contained in libraries, for example, synthetic or natural compounds in a combinatorial library. Numerous libraries are commercially available or can be readily produced; means for random and directed synthesis of a wide variety of organic compounds and biomolecules, including expression of randomized oligonucleotides, such as antisense oligonucleotides and oligopeptides, also are known. Alternatively, libraries of natural compounds in the form of bacterial, fungal, plant and animal extracts are available or can be readily produced. Additionally, natural or synthetically produced libraries and compounds are readily modified through conventional chemical, physical and biochemical means, and may be used to produce combinatorial libraries. Such libraries are useful for the screening of a large number of different compounds.

In some examples, the number of neutrophils is measured by counting the number of neutrophils in the blood of a subject or in the gut of a subject. Methods for counting neutrophils include manual counting (for example examining a sample (such as blood or tissue under a microscope) and counting the number of neutrophils or automated methods, such as flow cytometry. Neutrophils can be identified by staining techniques, including histological stains (such as hematoxylin and eosin), immunohistochemistry using neutrophil-specific antibodies or combinations of antibodies (such as anti-CD 11b, anti-CD68, anti-neutrophil elastase, anti-pANCA, or anti-MPO), or by detecting neutrophil-specific enzyme activity (such as chloroacetate esterase staining). In other examples, the number of neutrophils is measured using a label that is expressed under the control of a neutrophil-specific promoter (such as transgenic zebrafish expressing a fluorescent protein such as GFP under the control of the MPO promoter; Renshaw et al., Blood 108:3976-3978, 2006). In some examples, influx or numbers of neutrophils is measured using light sheet microscopy (see, *e.g.,* Baker et al., J. Microsc. 25:105-112, 2015), for example using light sheet microscopy to detect neutrophils expressing GFP under the control of the MPO promoter. A decrease in the number of neutrophil cells (such as an decrease of at least about 10%, about 20%, about 50%, about 80%, about 90%, about 1.5-fold, about 2-fold, about 3-fold, about 5-fold, about 10-fold or more) in zebrafish in the presence of one or more bacterial strains or test compounds as compared to in the absence of the one or more bacterial strains or test compounds indicates that the compound inhibits immune response or inflammation.

The following examples are provided to illustrate certain particular features and/or embodiments. These examples should not be construed to limit the disclosure to the particular features or embodiments described.

### Example 1

### Secreted A. veronii Anti-Inflammatory Factor

This example describes identification of a secreted factor from *A. veronii* with anti-inflammatory activity.

### Methods

All experiments performed with zebrafish were done according to protocols approved by the University of Oregon Institutional Animal Care and Use Committee. Conventionally-raised wild-type (AB x Tu strain) and *Tg(BACmpx:GFP)ⁱ¹¹⁴* (referred to as *mpx:GFP)* (Renshaw et al., Blood 108:3976-3978, 2006) were maintained as described (Westerfield, The Zebrafish Book, University of Oregon Press, Eugene, OR, 2000). The *mpx:GFP* zebrafish are transgenic for an insertion of EGFP at the MPO ATG start site.

Zebrafish embryos were derived germ free (GF) as previously described (Bates et al., Dev. Biol. 297:374-386, 2006), except the fish were soaked in 0.1% polyvinylpyrrolidone-iodine (PVP-I, Sigma-Aldrich, St. Louis, MO) for 2 minutes, washed three times in sterile embryo medium (EM), soaked in 0.003% bleach for 10 minutes, then washed in sterile EM. Subsequently, 15 GF embryos were transferred to sterile tissue culture flasks with 50 mL of EM. Mono-associated zebrafish were generated by inoculating the flask 4 days post fertilization (dpf) with 10⁶ colony forming units (CFU)/mL of bacteria. All manipulations to the GF flasks were performed under a class II A/B3 biological safety cabinet. The flasks were kept at 28°C until analysis of myeloperoxidase positive (MPO+) cells.

To create an inducible expression vector for the AP protein, the AP gene was amplified using the following primers: F1882NdeI, 5'-CGTACATATGATGAAAATGCACAACAAAGCGCTGC-3' (SEQ ID NO: 3) and R1882XhoI, 5'-CTGACTCGAGTTATCGCTTGTCAGCGGTGATCAG-3' (SEQ ID NO: 4) with NdeI and XhoI restriction sites included. The PCR amplification products and the vector (pET-21b) were digested with NdeI and XhoI (New England Biolabs, Ipswich, MA) and ligated using T4 ligase (NEB) following the manufacturer's protocol. Subsequently, the ligated product was transformed into BL21 *E. coli.* To induce AP expression, *E. coli* was treated with 0.1 M IPTG in early exponential phase and was allowed to grow and produce protein for 2 hours. This resulted in a supernatant that was dominated by AP (FIG. 8).

After *A. veronii* was grown over night to stationary phase and after protein induction in *E. coli,* concentrated cell-free supernatant (CFS) was prepared. The 50-mL cultures were centrifuged at 7000 x g for 10 minutes at 4°C. Subsequently, the supernatant was filtered through a 0.22-µm sterile tube top filter (Corning Inc., NY). The sterile supernatant was concentrated at 4°C for 1 hour at 3000 x g with a centrifugal device that has a 10 kDa weight cut off (Pall Life Sciences). The concentration of the supernatant was determined with a Nanodrop and inoculated into the flasks for a final concentration of 500 ng/mL.

MPO+ cell analysis was performed on 6 dpf. The *mpx:GFP* zebrafish were anesthetized in Tricaine and mounted in 4% methylcellulose. Subsequently, their guts were sterilely dissected. The number of GFP-positive cells was quantified visually for each fish.

### Results

In order to identify factors secreted by zebrafish gut microbiota that potentially alter innate immune responses, zebrafish larvae were reared conventionally (CV), germ-free (GF), in the presence of *A. veronii,* or in the presence of a mutant *A. veronii* strain lacking the type II secretion system (ΔT2SS; Maltz and Graf, Appl. Environ. Microbiol. 77:597-603, 2011). One strain of *A. veronii*, ZF01, induced significantly more neutrophil influx than the genetically similar *A. veronii* Hm21. This result suggested that *A. veronii* Hm21 secreted a factor that reduced neutrophil influx in the intestine and Hm21 was selected for further investigation.

The type II secretion system (T2SS) is the main terminal branch of the general secretion pathway and is involved in secretion of proteins including proteases, cellulases, pectinases, phospholipases, lipases, and toxins. The ΔT2SS *A. veronii* strain has a reduced number of secreted proteins compared to strains with an intact T2SS system. Zebrafish raised in the presence of ΔT2SS *A. veronii* Hm21 had a greater neutrophil response than those raised GF or in the presence of wild-type *A. veronii* (FIG. 1). CFS from wild-type *A. veronii* rescued the low neutrophil infiltration phenotype in zebrafish raised with ΔT2SS *A. veronii* (FIG. 2).

Mass spectrometry of CFS from wild type and ΔT2SS *A. veronii* revealed significant increases in several secreted proteins (Table 1). CFS from wild-type *A. veronii* was fractionated by ammonium sulfate precipitation, and fractions were tested for their ability to stimulate neutrophil response (FIG. 3). The activity was concentrated in one fraction. The fractions were analyzed by SDS-PAGE and the active fraction was found to contain two major bands - one of about 55 kD and one of about 35 kD (FIG. 4). The molecular weight of the proteins with increased amounts in wild-type versus ΔT2SS *A. veronii* was considered (Table 1) to select candidate anti-inflammatory proteins.

**Table 1. Molecular weight of proteins increased in WT A. veronii as compared to ΔT2SS A. veronii**

| **MW (kD)** | **Protein** |
|---|---|
| 109.316 | Metalloprotease stce OS=Aeromonas veronii (strain B565) |
| 51.919 | Chitin-binding protein, carbohydrate-binding module family (CBP) |
| 63.724 | Protease OS=Aeromonas veronii (strain B565) |
| 91.892 | Putative trimethylamine-N-oxide reductase 1 |
| 69.583 | Chitinase |
| 130.48 | Putative uncharacterized protein |
| 103.622 | Collagenase family |
| 53.921 | Aeromonas virulence factor (hemolysin) |
| 66.694 | Serine protease Ahe2 |
| 93.066 | Chitinase A |
| 145.483 | Pullulanase |
| 61.732 | UshA protein |
| 107.821 | Chitinase |
| 33.386 | Putative uncharacterized protein (called 1882 or AP) |
| 79.011 | Glycoside hydrolase family 18 |
| 70.456 | Twin-arginine translocation pathway signal |
| 78.444 | Predicted extracellular nuclease |
| 92.107 | Chitinase 92 |
| 73.333 | 2',3'-cyclic-nucleotide 2'-phosphodiesterase |

To test the activity of candidate *A. veronii* proteins, AP and CBP were each cloned and expressed in *E. coli.* The *E. coli* genome does not contain homologues of AP or CBP. CFS from the recombinant *E. coli* was applied to GF-reared zebrafish. Neutrophil influx was decreased by CFS from *E. coli* expressing AP; however CFS from *E. coli* expressing CBP had no effect on neutrophil influx (FIG. 5).

### Example 2

### Effect of AP on Response to Infection

This example describes the effect of AP on response of zebrafish to *Vibrio* infection.

Zebrafish were raised as described in Example 1, either GF or mono-associated with a *Vibrio* species isolated from zebrafish gut. After 48 hours of *Vibrio* infection (inoculated at 10⁶ cfu/ml), CFS from *E. coli* expressing AP was inoculated in the culture at 500 ng/ml. MPO positive cells in the gut were measured as described in Example 1.

Infection with *Vibrio* increased the neutrophil influx in zebrafish gut as compared to GF zebrafish (FIG. 6). Treatment of zebrafish infected with *Vibrio* with CFS from *E. coli* expressing anti-inflammatory protein AP reduced neutrophil influx in response to *Vibrio* infection to levels comparable to that in GF zebrafish (FIG. 6). CFS from *E. coli* carrying a control plasmid had no effect on neutrophil influx.

### Example 3

### Effect of AP in a Zebrafish Model of Colitis

This example describes the effect of AP in a zebrafish mutant (*sox10*) that is a model of colitis.

Zebrafish *sox10* mutant fails to differentiate enteric neurons, resulting in a lack of rhythmic peristaltic activity (Renshaw et al., Blood 108:3976-3978, 2006) and is a model for colitis. These mutants had significantly higher bacterial loads than their wild-type siblings (FIG. 9A). 16S rRNA profiling of the *sox10* gut microbiota revealed a reduced diversity (alpha diversity) compared to the microbiota from their wild-type siblings (FIG. 9B), which is similar to the types of microbial dysbiosis observed in ulcerative colitis (Renshaw et al., Blood 108:3976-3978, 2006). Furthermore, these mutants had a significantly increased level of neutrophils in their intestine (FIG. 9C), a phenotype that was transferred to wild-type zebrafish upon inoculation of germ-free fish with the microbiota from *sox10* fish (FIG. 9D), similar to transmissible colitis mouse models (Zenewicz et al., J. Immunol. 190:5306-5312, 2013; Garrett et al., Cell 131:33-45, 2007). These data demonstrated that the *sox10* fish can be used as a model for dysbiosis and transmissible intestinal inflammation. In addition, treating *sox10* mutants with AP significantly reduced neutrophil influx to the intestine (FIG. 10).

### Example 4

### Effect of AP Knockout Strain in Zebrafish

This example describes a bacterial strain that does not express AP and its effect in zebrafish.

An *Aeromonas* AP knockout strain was produced using homologous recombination to replace the AP gene with a chloramphenicol resistance cassette (cm^{R}) (*A. veronii Δap::cm^{R}*)*.* The cm^{R} was amplified from the pKD3 plasmid (GenBank accession number AY048742) using the primers 1882cm.Mid5 (5' GCGACAGCAAGGAATAAAAACTC; SEQ ID NO: 5) and 1882cm.Mid3 (5' CACCCCTGCCGTTAGCTGCTTAT; SEQ ID NO: 6). These primers include sequence that overlaps with Hm21 genome surrounding the AP gene sequence. An approximately 1000 base pair region upstream and downstream of the AP gene was amplified by PCR using the following primers: Cm1882.up3 (5'CTAAGGAGGATATTCATATGCAT; SEQ ID NO: 7), 1882.up5 (5' GATGGTCTGGGTATTGCCGTTG; SEQ ID NO: 8), cm1882.dn5 (5' CGAAGCAGCTCCAGCCTACACA; SEQ ID NO: 9), and 1882.dn3 (5' GCTGTTCGTCATCGATCGGCGC; SEQ ID NO: 10). The amplification products were put together using the three products as a template and the 1882.up5 and 1882.dn3 primers. Subsequently, the piece was ligated into the pDMS197 plasmid (Edwards et al., Gene 207:149-157, 1998). The resulting plasmid was transformed into the *E. coli* λpir+ SM10 strain, which was used to mate at 30°C for 4 hours with Aeromonas Hm21. *Aeromonas* Hm21 ΔAP were selected by resistance to chloramphenicol and confirm by PCR.

This mutant did not have a growth defect *in vitro* and colonized the zebrafish to wild-type levels. Infection of zebrafish with an *A. veronii* Hm21 whose AP was knocked out (ΔAP) induced more inflammation than wild-type *A. veronii* Hm21 (FIG. 11).

### Comparative Example 5

### Methods of Screening for Modulators of Immune Response or Inflammation

This example describes particular methods that can be used for screening for modulators of immune response or inflammation utilizing transgenic zebrafish that express GFP under the control of the MPO promoter. One skilled in the art will appreciate that methods that deviate from these specific methods can also be used to successfully screen for modulators of immune response or inflammation.

Protocol for screening anti-inflammatory protein:
Day 1: Set up *mpo*:GFP fish to cross naturally. Use dividers to prevent egg laying until the morning.
Day 2 (0 dpf):
   1. By 9:00 am, move natural crosses into tanks with fresh water and pull dividers to allow fish to mate. Prepare antibiotic EM to collect eggs (100 µg/mL ampicillin, 5 µg/mL kanamycin, and 250 µg/ml amphotericin B, sterile filtered). Collect eggs in antibiotic EM and place in 30°C incubator until they reach shield stage.
   2. Move embryos into sterile 50-mL beaker. Wash embryos 3x in sterile EM. Immerse embryos in 0.1% PVP-I solution for 2 minutes. Rinse 3x in sterile EM. Transfer embryos to a new sterile 50-mL beaker and immerse in 0.003% bleach for 20 minutes. Pour off bleach and rinse 3x in sterile EM. Transfer 15 embryos into 50-mL sterile cell culture flasks with 15-mL sterile EM.
Day 5 (3 dpf):
   1. Start overnight bacterial cultures. Start 50-mL cultures of any of the bacteria whose CFS you want to test for anti-inflammatory activity. Additionally start cultures that you need to induce inflammation, for example *A. veronii* or *Vibrio.* I estimate that a person could reasonably test 8 to 10 different bacterial CFS preparations in one experiment.
Day 6 (4 dpf):
   1. Follow CFS preparation protocol listed above.
   2. Visually check GF fish flasks for bacteria.
   3. Inoculate flasks with 10⁶ cfu/ml bacteria (either *A. veronii* or *Vibrio)* and in some also include 500 ng/ml concentrated CFS. For each experiment, include one flask with no additional protein as a control and a GF flask as a control. Collect 1-mL of flask water before inoculation to plate and confirm that flasks were germ free at the start of the experiment.
Day 8 (6 dpf):
   1. Check the plates with the inoculation water to ensure the flasks were germ-free before you started.
   2. One flask at a time, add tricaine to the fish to anesthetize them. Rub 5% methylcellulose on a microscope slide. Use a glass pipette to carefully pull each fish out of the flask and place it on the prepared slide. Use the pipette to pull off any excess EM from the slide.
   3. Under a dissecting microscope, use dissecting needles to pull the gut out of the fish, keeping it intact. Using a fluorescence microscope, visualize the GFP with a 395-nm light and count the number of neutrophils that are associated with the gut. Repeat steps 2 and 3 for 10-15 fish per each CFS treatment.

### Example 6

### Testing of AP in Zebrafish Models of Disease

This example describes particular methods that can be used to test the effect of AP on inflammation in zebrafish models of gut disease. However, one skilled in the art will appreciate that methods that deviate from these specific methods can also be used to successfully test the effect of AP on inflammation in zebrafish.

Zebrafish are treated with AP (for example, purified protein or CFS from *E. coli* expressing AP) and one or more markers of inflammation in the gut are determined. A reduction of one or more markers of inflammation in fish treated with AP as compared to control (untreated fish) indicates that AP decreases inflammation in the zebrafish gut.

In one example, a zebrafish model of Hirschsprung disease is used. These zebrafish have a mutation in a transcription factor, which results in a lack of peristalsis and a significant increase in gut inflammation over WT fish. This line is crossed with *mpo*:GFP zebrafish. The fish are raised conventionally and treated with the AP-enriched CFS (*e.g*., 500-1000 ng/mL) on 4 dpf. On 6 dpf the guts are dissected and the number of infiltrating neutrophils in the gut is counted (for example, by detecting GFP). A reduction in neutrophil influx to the gut in AP-treated fish compared to the untreated fish indicates that this protein reduces inflammation in a model of Hirschsprung disease.

In another example, larval zebrafish exposed to trinitrobenzene sulfonic acid (TNBS) have impaired intestinal homeostasis and inflammation that models what is observed in human inflammatory bowel disease (IBD). To test the activity of AP in this model, conventionally raised 3 dpf *mpo*:GFP zebrafish are placed in groups of 15 fish in 15-mL EM and TNBS is added to a final concentration of 100 µg/mL. On 4 dpf the fish are treated with 500 ng/mL to 1000 ng/mL AP-enriched CFS from the induced *E. coli* expressing anti-inflammatory protein AP. On 6 dpf the guts are dissected and the number of infiltrating neutrophils in the gut is counted (for example, by detecting GFP). A reduction in neutrophil influx to the gut in AP-treated fish compared to the untreated fish indicates that this protein reduces inflammation in a model of IBD.

### Example 7

### Testing of AP in Mice

This example describes particular methods that can be used to test the effect of AP on inflammation in mice. However, one skilled in the art will appreciate that methods that deviate from these specific methods can also be used to successfully test the effect of AP on inflammation in mice

Adult mice are placed on three, 3-day cycles of 3% Dextran Sulfate Sodium (DSS) (Whittem et al., J. Vis. Exp. 35:6-8, 2010) with seven days of recovery between each cycle. A baseline weight for each mouse is obtained prior to DSS treatment and mice are weighed regularly during the experiment. As a positive control, additional mice are administered mouse IL-1ra (Sigma), which has been shown to alleviate inflammatory symptoms and neutrophil infiltration (reviewed in Rolig et al., Infect. Immun. 81:1382-1389, 2013). Treatment with AP is administration of 50-1000 ng/ml of purified AP added to the drinking water during the DSS treatment cycles. After the final recovery period, each mouse is weighed, sacrificed, and necropsied. In necropsy, the colon is removed and the length is documented, then fixed in 4% paraformaldehyde. Samples are embedded in paraffin, mounted, sectioned, and stained with hematoxylin and eosin for histologic analysis. Pathology is scored, including inflammation severity and epithelial cell integrity in the experimental and control colons (for example, in a double blind fashion). The ability of AP to alleviate inflammation in the colon is compared to exogenous mouse IL-1ra. Specific markers of inflammation are also evaluated, for example, Ly6B.2 (neutrophils), F4/80 (macrophages), and Ki67 (proliferation). Effectiveness of AP in mice may be indicated by decreased weight loss, increased colon length, and/or decreased pathologic and histologic markers of inflammation compared to mice treated with DSS alone (no AP treatment).

### Example 8

### Method of Treating or Inhibiting Inflammation

This example describes particular methods that can be used to treat or inhibit inflammation in a subject. However, one skilled in the art will appreciate that methods that deviate from these specific methods can also be used to successfully treat or inhibit inflammation in a subject.

Based upon the teaching disclosed herein, inflammation or an inflammatory disease can be treated or inhibited by administering an effective amount of a composition including an Aeromonas anti-inflammatory protein, a nucleic acid encoding the protein, or a preparation including bacteria that produce the protein to a subject with inflammation or an inflammatory disease.

In an example, a subject with an inflammatory disease is identified and selected for treatment. For example, a subject diagnosed with inflammatory bowel disease may be selected for treatment. Following subject selection, an effective dose of the composition or preparation including the anti-inflammatory protein, nucleic acid, or bacteria described above is administered to the subject. The amount of the composition or preparation administered to prevent, reduce, inhibit, and/or treat inflammation or an inflammatory disease depends on the subject being treated, the severity of the disorder, and the manner of administration of the composition. Ideally, an effective amount of an agent is the amount sufficient to prevent, reduce, and/or inhibit, and/or treat the condition (*e.g*., inflammatory disease) in a subject without causing substantial adverse effects in the subject.

In one specific example, an anti-inflammatory protein (such as SEQ ID NO: 1) or a fragment thereof (such as amino acids 148-176 of SEQ ID NO: 1) is administered to a subject. For example, an anti-inflammatory protein is administered to a subject at about 1 mg to 1 g daily. In another example, an anti-inflammatory protein is administered at about 1 mg to 1 g biweekly or weekly. In further examples, a nucleic acid encoding an anti-inflammatory protein (such as SEQ ID NO: 2) is administered to a subject at about 1 mg to 1 g daily, biweekly, or weekly. An appropriate dose can be selected by a skilled clinician based on the subject, the condition being treated and other factors.

Subjects are monitored by methods known to those skilled in the art to determine responsiveness of the inflammation or inflammatory disease treatment. For example, if the condition is inflammatory bowel disease, the symptoms of the subject are monitored, for example using the Crohn's Disease Activity Index or the Harvey-Bradshaw Index. It is contemplated that additional agents can be administered, such as additional anti-inflammatory agents in combination with or following treatment with the *Aeromonas* anti-inflammatory protein.

In view of the many possible embodiments to which the principles of the disclosure may be applied, it should be recognized that the illustrated embodiments are only examples and should not be taken as limiting the scope of the invention. Rather, the scope of the invention is defined by the following claims.

## Claims

1. An isolated polypeptide for use in a method of treating or inhibiting inflammation in a subject, which polypeptide:
comprises an amino acid sequence having at least 80% sequence identity to the amino acid sequence of SEQ ID NO: 1 or is an isolated polypeptide having at least 80% sequence identity to a fragment of SEQ ID NO: 1;
comprises an amino acid sequence at least 90% identical to the amino acid sequence set forth as SEQ ID NO: 1 or the fragment thereof;
comprises an amino acid sequence at least 95% identical to the amino acid sequence set forth as SEQ ID NO: 1 or the fragment thereof;
comprises the amino acid sequence set forth as SEQ ID NO: 1;
consists of the amino acid sequence set forth as SEQ ID NO: 1 or a fragment thereof;
is encoded by a nucleic acid comprising a nucleotide sequence at least 80% identical to the nucleic acid sequence set forth as SEQ ID NO: 2 or a fragment of SEQ ID NO: 2;
is encoded by a nucleic acid comprising a nucleotide sequence at least 90% identical to the nucleic acid sequence set forth as SEQ ID NO: 2 or a fragment of SEQ ID NO: 2;
is encoded by a nucleic acid comprising a nucleotide sequence at least 95% identical to the nucleic acid sequence set forth as SEQ ID NO: 2 or a fragment of SEQ ID NO: 2;
is encoded by a nucleic acid comprising the nucleic acid sequence set forth as SEQ ID NO: 2 or a fragment of SEQ ID NO: 2; or
is encoded by a nucleic acid consisting of the nucleic acid sequence set forth as SEQ ID NO: 2 or a fragment thereof.

2. The isolated polypeptide for use of claim 1, wherein the fragment of SEQ ID NO: 1 comprises or consists of amino acids 23-313 of SEQ ID NO: 1, amino acids 103-176 of SEQ ID NO: 1, or amino acids 148-176 of SEQ ID NO: 1.

3. The isolated polypeptide for the use of claim 1 or claim 2, wherein the inflammation in the subject comprises an inflammatory disease.

4. The isolated polypeptide for the use of claim 3, wherein the inflammatory disease comprises rheumatoid arthritis, osteoarthritis, inflammatory lung disease, inflammatory bowel disease, Hirschsprung's associated enterocolitis, pelvic inflammatory disease, periodontal disease, polymyalgia rheumatica, atherosclerosis, systemic lupus erythematosus, systemic sclerosis, Sjogren's Syndrome, asthma, allergic rhinitis, psoriasis, irritable bowel syndrome, necrotizing enterocolitis, or atopy.

5. The isolated polypeptide for the use of any one of claims 1 to 4, wherein the amount of the polypeptide administered to the subject is about 1 mg to about 5 g.

6. The isolated polypeptide for the use of any one of claims 1 to 5, wherein the effective amount of the polypeptide is administered to the subject daily, weekly, or monthly.

7. The isolated polypeptide for the use of any one of claims 1 to 6, wherein the method further comprises administering one or more additional anti-inflammatory therapies to the subject.

8. The isolated polypeptide for the use of any one of claims 1 to 7, wherein the polypeptide retains a function of the anti-inflammatory protein of SEQ ID NO: 1, said function being selected from: decreasing the number or activation of neutrophils; and reducing or inhibiting inflammation in a subject.

9. An isolated polynucleotide which encodes a polypeptide as defined in claim 1, 2 or 8, or which:
comprises a nucleotide sequence at least 80% identical to the nucleic acid sequence set forth as SEQ ID NO: 2 or a fragment thereof;
comprises a nucleotide sequence at least 90% identical to the nucleic acid sequence set forth as SEQ ID NO: 2 or a fragment thereof;
comprises a nucleotide sequence at least 95% identical to the nucleic acid sequence set forth as SEQ ID NO: 2 or a fragment thereof;
comprises the nucleic acid sequence set forth as SEQ ID NO: 2 or a fragment thereof; or
consists of the nucleic acid sequence set forth as SEQ ID NO: 2 or a fragment thereof;
a recombinant vector comprising said polynucleotide operably linked to a heterologous promoter, or
a transformed cell comprising said recombinant vector,
for use in a method of treating or inhibiting inflammation in a subject.

10. The isolated polynucleotide, recombinant vector, or transformed cell for the use of claim 9, wherein the inflammation in the subject comprises an inflammatory disease.

11. The isolated polynucleotide, recombinant vector, or transformed cell for the use of claim 10, wherein the inflammatory disease comprises rheumatoid arthritis, osteoarthritis, inflammatory lung disease, inflammatory bowel disease, Hirschsprung's associated enterocolitis, pelvic inflammatory disease, periodontal disease, polymyalgia rheumatica, atherosclerosis, systemic lupus erythematosus, systemic sclerosis, Sjogren's Syndrome, asthma, allergic rhinitis, psoriasis, irritable bowel syndrome, necrotizing enterocolitis, or atopy.

## Patentansprüche

1. Isoliertes Polypeptid zur Verwendung in einem Verfahren zur Behandlung oder Hemmung von Entzündung bei einem Individuum, wobei das Polypeptid:
eine Aminosäuresequenz mit zumindest 80 % Sequenzidentität mit der Aminosäuresequenz der SEQ ID No: 1 umfasst oder ein isoliertes Polypeptid mit zumindest 80 % Sequenzidentität mit einem Fragment der SEQ ID No: 1 ist;
eine Aminosäuresequenz mit zumindest 90 % Identität mit der Aminosäuresequenz, die als SEQ ID No: 1 dargelegt ist, oder dem Fragment davon umfasst;
eine Aminosäuresequenz mit zumindest 95 % Identität mit der Aminosäuresequenz, die als SEQ ID No: 1 dargelegt ist, oder dem Fragment davon umfasst;
die Aminosäuresequenz, die als SEQ ID No: 1 dargelegt ist, umfasst;
aus der Aminosäuresequenz, die als SEQ ID No: 1 dargelegt ist, oder einem Fragment davon besteht;
durch eine Nucleinsäure, die eine Nucleotidsequenz mit zumindest 80 % Identität mit der Nucleinsäuresequenz, die als SEQ ID No: 2 dargelegt ist, oder einem Fragment der SEQ ID No: 2 umfasst, kodiert wird;
durch eine Nucleinsäure, die eine Nucleotidsequenz mit zumindest 90 % Identität mit der Nucleinsäuresequenz, die als SEQ ID No: 2 dargelegt ist, oder einem Fragment der SEQ ID No: 2 umfasst, kodiert wird;
durch eine Nucleinsäure, die eine Nucleotidsequenz mit zumindest 95 % Identität mit der Nucleinsäuresequenz, die als SEQ ID No: 2 dargelegt ist, oder einem Fragment der SEQ ID No: 2 umfasst, kodiert wird;
durch eine Nucleinsäure, die die Nucleotidsequenz, die als SEQ ID No: 2 dargelegt ist, oder ein Fragment der SEQ ID No: 2 umfasst, kodiert wird; oder
durch eine Nucleinsäure, die aus der Nucleotidsequenz, die als SEQ ID No: 2 dargelegt ist, oder einem Fragment der SEQ ID No: 2 besteht, kodiert wird.

2. Isoliertes Polypeptid zur Verwendung nach Anspruch 1, wobei das Fragment der SEQ ID No: 1 die Aminosäuren 23 bis 313 der SEQ ID No: 1, die Aminosäuren 103 bis 176 der SEQ ID No: 1 oder die Aminosäuren 148 bis 176 der SEQ ID No: 1 umfasst oder aus diesen besteht.

3. Isoliertes Polypeptid zur Verwendung nach Anspruch 1 oder Anspruch 2, wobei die Entzündung bei einem Individuum eine Entzündungserkrankung umfasst.

4. Isoliertes Polypeptid zur Verwendung nach Anspruch 3, wobei die Entzündungserkrankung rheumatoide Arthritis, Osteoarthritis, entzündliche Lungenerkrankung, chronisch-entzündliche Darmerkrankung, Hirschsprung-assoziierte Enterokolitis, entzündliche Beckenerkrankung, Parodontose, Polymyalgia rheumatica, Atherosklerose, systemischen Lupus erythematodes, systemische Sklerose, Sjögren-Syndrom, Asthma, allergische Rhinitis, Psoriasis, Reizdarmsyndrom, nekrotisierende Enterokolitis oder Atopie umfasst.

5. Isoliertes Polypeptid zur Verwendung nach einem der Ansprüche 1 bis 4, wobei die Menge an Polypeptid, die dem Individuum verabreicht wird, etwa 1 mg bis etwa 5 g beträgt.

6. Isoliertes Polypeptid zur Verwendung nach einem der Ansprüche 1 bis 5, wobei die wirksame Menge an Polypeptid dem Individuum täglich, wöchentlich oder monatlich verabreicht wird.

7. Isoliertes Polypeptid zur Verwendung nach einem der Ansprüche 1 bis 6, wobei das Verfahren weiters das Verabreichen einer oder mehrerer zusätzlicher entzündungshemmender Therapien an das Individuum umfasst.

8. Isoliertes Polypeptid zur Verwendung nach einem der Ansprüche 1 bis 7, wobei das Polypeptid eine Funktion des entzündungshemmenden Proteins der SEQ ID No: 1 beibehält, wobei die Funktion aus den folgenden ausgewählt ist: Senken der Anzahl oder Aktivierung von Neutrophilen; und Verringern oder Hemmen der Entzündung bei einem Individuum.

9. Isoliertes Polynucleotid, das für ein Polypeptid wie definiert in Anspruch 1, 2 oder 8 kodiert oder das
eine Nucleotidsequenz, die zumindest 80 % Identität mit der Nucleinsäuresequenz, die in SEQ ID No: 2 dargelegt ist, oder einem Fragment davon aufweist, umfasst;
eine Nucleotidsequenz, die zumindest 90 % Identität mit der Nucleinsäuresequenz, die in SEQ ID No: 2 dargelegt ist, oder einem Fragment davon aufweist, umfasst;
eine Nucleotidsequenz, die zumindest 95 % Identität mit der Nucleinsäuresequenz, die in SEQ ID No: 2 dargelegt ist, oder einem Fragment davon aufweist, umfasst;
die Nucleotidsequenz, die in SEQ ID No: 2 dargelegt ist, oder ein Fragment davon umfasst; oder
aus der Nucleotidsequenz, die in SEQ ID No: 2 dargelegt ist, oder einem Fragment davon besteht;
rekombinanter Vektor, der das Polynucleotid, das operabel an einen heterologen Promotor gebunden ist, umfasst, oder
transformierte Zelle, die den rekombinanten Vektor umfasst,
zur Verwendung in einem Verfahren zur Behandlung oder Hemmung von Entzündung bei einem Individuum.

10. Isoliertes Polynucleotid, rekombinanter Vektor oder transformierte Zelle zur Verwendung nach Anspruch 9, wobei die Entzündung bei dem Individuum eine Entzündungserkrankung umfasst.

11. Isoliertes Polypeptid, rekombinanter Vektor oder transformierte Zelle zur Verwendung nach Anspruch 10, wobei die Entzündungserkrankung rheumatoide Arthritis, Osteoarthritis, entzündliche Lungenerkrankung, chronisch-entzündliche Darmerkrankung, Hirschsprung-assoziierte Enterokolitis, entzündliche Beckenerkrankung, Parodontose, Polymyalgia rheumatica, Atherosklerose, systemischen Lupus erythematodes, systemische Sklerose, Sjögren-Syndrom, Asthma, allergische Rhinitis, Psoriasis, Reizdarmsyndrom, nekrotisierende Enterokolitis oder Atopie umfasst.

## Revendications

1. Polypeptide isolé destiné à être utilisé dans un procédé de traitement ou d'inhibition d'une inflammation chez un sujet, lequel polypeptide :
comprend une séquence d'acides aminés présentant au moins 80 % d'identité de séquence avec la séquence d'acides aminés de SEQ ID NO: 1 ou est un polypeptide isolé présentant au moins 80 % d'identité de séquence avec un fragment de SEQ ID NO: 1 ;
comprend une séquence d'acides aminés au moins 90 % identique à la séquence d'acides aminés décrite en tant que SEQ ID NO: 1 ou au fragment de celle-ci ;
comprend une séquence d'acides aminés au moins 95 % identique à la séquence d'acides aminés décrite en tant que SEQ ID NO: 1 ou au fragment de celle-ci ;
comprend la séquence d'acides aminés décrite en tant que SEQ ID NO: 1 ;
consiste en la séquence d'acides aminés décrite en tant que SEQ ID NO: 1 ou un fragment de celle-ci ;
est codé par un acide nucléique comprenant une séquence de nucléotides au moins 80 % identique à la séquence d'acide nucléique décrite en tant que SEQ ID NO: 2 ou à un fragment de SEQ ID NO: 2 ;
est codé par un acide nucléique comprenant une séquence de nucléotides au moins 90 % identique à la séquence d'acide nucléique décrite en tant que SEQ ID NO: 2 ou à un fragment de SEQ ID NO: 2 ;
est codé par un acide nucléique comprenant une séquence de nucléotides au moins 95 % identique à la séquence d'acide nucléique décrite en tant que SEQ ID NO: 2 ou à un fragment de SEQ ID NO: 2 ;
est codé par un acide nucléique comprenant la séquence d'acide nucléique décrite en tant que SEQ ID NO: 2 ou un fragment de SEQ ID NO: 2 ; ou
est codé par un acide nucléique consistant en la séquence d'acide nucléique décrite en tant que SEQ ID NO: 2 ou un fragment de celle-ci.

2. Polypeptide isolé destiné à être utilisé selon la revendication 1, dans lequel le fragment de SEQ ID NO: 1 comprend ou consiste en les acides aminés 23-313 de SEQ ID NO: 1, les acides aminés 103-176 de SEQ ID NO: 1, ou les acides aminés 148-176 de SEQ ID NO: 1.

3. Polypeptide isolé destiné à être utilisé selon la revendication 1 ou la revendication 2, où l'inflammation chez le sujet comprend une maladie inflammatoire.

4. Polypeptide isolé destiné à être utilisé selon la revendication 3, où la maladie inflammatoire comprend la polyarthrite rhumatoïde, l'arthrose, une maladie pulmonaire inflammatoire, une maladie intestinale inflammatoire, l'entérocolite de Hirschsprung, une maladie inflammatoire pelvienne, une maladie parodontale, la polymyalgie rhumatismale, l'athérosclérose, le lupus érythémateux systémique, la sclérodermie systémique, le syndrome de Sjögren, l'asthme, la rhinite allergique, le psoriasis, le syndrome du côlon irritable, l'entérocolite nécrosante, ou l'atopie.

5. Polypeptide isolé destiné à être utilisé selon l'une quelconque des revendications 1 à 4, où la quantité du polypeptide administrée au sujet est environ 1 mg à environ 5 g.

6. Polypeptide isolé destiné à être utilisé selon l'une quelconque des revendications 1 à 5, où la quantité efficace du polypeptide est administrée au sujet tous les jours, toutes les semaines, ou tous les mois.

7. Polypeptide isolé destiné à être utilisé selon l'une quelconque des revendications 1 à 6, où le procédé comprend en outre l'administration d'une ou de plusieurs thérapies anti-inflammatoires supplémentaires au sujet.

8. Polypeptide isolé destiné à être utilisé selon l'une quelconque des revendications 1 à 7, où le polypeptide conserve une fonction de la protéine anti-inflammatoire de SEQ ID NO: 1, ladite fonction étant sélectionnée parmi : une réduction du nombre ou de l'activation des neutrophiles ; et une réduction ou une inhibition de l'inflammation chez un sujet.

9. Polynucléotide isolé qui code pour un polypeptide tel que défini dans la revendication 1, 2 ou 8, ou qui :
comprend une séquence de nucléotides au moins 80 % identique à la séquence d'acide nucléique décrite en tant que SEQ ID NO: 2 ou à un fragment de celle-ci ;
comprend une séquence de nucléotides au moins 90 % identique à la séquence d'acide nucléique décrite en tant que SEQ ID NO: 2 ou à un fragment de celle-ci ;
comprend une séquence de nucléotides au moins 95 % identique à la séquence d'acide nucléique décrite en tant que SEQ ID NO: 2 ou à un fragment de celle-ci ;
comprend la séquence d'acide nucléique décrite en tant que SEQ ID NO: 2 ou un fragment de celle-ci ; ou
consiste en la séquence d'acide nucléique décrite en tant que SEQ ID NO: 2 ou un fragment de celle-ci ;
un vecteur recombinant comprenant ledit polynucléotide en liaison fonctionnelle avec un promoteur hétérologue, ou
une cellule transformée comprenant ledit vecteur recombinant,
destiné à être utilisé dans un procédé de traitement ou d'inhibition d'une inflammation chez un sujet.

10. Polynucléotide isolé, vecteur recombinant, ou cellule transformée destiné(e) à être utilisé(e) selon la revendication 9, où l'inflammation chez le sujet comprend une maladie inflammatoire.

11. Polynucléotide isolé, vecteur recombinant, ou cellule transformée destiné(e) à être utilisé(e) selon la revendication 10, où la maladie inflammatoire comprend la polyarthrite rhumatoïde, l'arthrose, une maladie pulmonaire inflammatoire, une maladie intestinale inflammatoire, l'entérocolite de Hirschsprung, une maladie inflammatoire pelvienne, une maladie parodontale, la polymyalgie rhumatismale, l'athérosclérose, le lupus érythémateux systémique, la sclérodermie systémique, le syndrome de Sjögren, l'asthme, la rhinite allergique, le psoriasis, le syndrome du côlon irritable, l'entérocolite nécrosante, ou l'atopie.
